# EUROPÄISCHE PATENTSCHRIFT

(11) **EP 2 556 856 B1**
(45) Veröffentlichungstag und Bekanntmachung des Hinweises auf die Patenterteilung: **25.02.2015**
(21) Anmeldenummer: 12189433.1
(22) Anmeldetag: 09.12.2008
(51) Int. Cl.: A61P 9/00, A61P 3/10, A61K 31/519, C07D 215/54, C07D 221/04, C07D 471/04

(54) **6-Cyano-substituierte Pyrido[2,3-d]pyrimidine als Adenosin Rezeptor Liganden zur Behandlung von Kardiovaskulären Erkrankungen**
6-Cyano-substituted Pyrido[2,3-d]pyrimidines as Adenosin Rezeptor Ligands for the Treatment of Cardiovascular Diseases
6-Carbonitriles-pyrido[2,3-d]pyrimidines en tant que ligand pour le récepteur Adenosin pour le traitement des maladies cardiovasculaires

(30) Priorität: 20.12.2007 DE 102007061764
(43) Veröffentlichungstag der Anmeldung: 13.02.2013
(62) Teilanmeldung aus: 08864116.2
(73) Patentinhaber: Bayer Intellectual Property GmbH, 40789 Monheim (DE)
(72) Erfinder: Nell, Peter, 94062 Woodside (US); Vakalopoulos, Alexandros, 40721 Hilden (DE); Süssmeier, Frank, 80992 München (DE); Albrecht-Küpper, Barbara, 42489 Wülfrath (DE); Zimmermann, Katja, 40489 Düsseldorf (DE); Keldenich, Joerg, 42113 Wuppertal (DE); Meiblom, Daniel, 42113 Wuppertal (DE)
(74) Vertreter: BIP Patents

(56) Entgegenhaltungen:
- WO-A2-01/25210
- US-A1- 2006 014 764
- BULICZ J ET AL: "Synthesis and pharmacology of pyrido[2,3-d]pyrimidinediones bearing polar substituents as adenosine receptor antagonists", BIOORGANIC & MEDICINAL CHEMISTRY, PERGAMON, GB, Bd. 14, Nr. 8, 15. April 2006 (2006-04-15) , Seiten 2837-2849, XP027992543, ISSN: 0968-0896 [gefunden am 2006-04-15]

## Beschreibung

Die vorliegende Anmeldung betrifft neue substituierte anellierte Cyanopyridin-Derivate, Verfahren zu ihrer Herstellung, die beanspruchten Verbindungen zur Verwendung in der Behandlung und/oder Prävention von Krankheiten sowie ihre Verwendung zur Herstellung von Arzneimitteln zur Behandlung und/oder Prävention von Krankheiten, vorzugsweise zur Behandlung und/oder Prävention von kardiovaskulären Erkrankungen.

Adenosin, ein Purin-Nukleosid, ist in allen Zellen vorhanden und wird unter einer Vielzahl von physiologischen und pathophysiologischen Stimuli freigesetzt. Adenosin entsteht intrazellulär beim Abbau von Adenosin-5'-monophosphat (AMP) und S-Adenosylhomocystein als Zwischenprodukt, kann jedoch aus der Zelle freigesetzt werden und übt dann durch Bindung an spezifische Rezeptoren Funktionen als hormonähnliche Substanz oder Neurotransmitter aus.

Unter normoxischen Bedingungen ist die Konzentration des freien Adenosin im Extrazellulärraum sehr niedrig. Die extrazelluläre Konzentration von Adenosin erhöht sich in den betroffenen Organen jedoch dramatisch unter ischämischen bzw. hypoxischen Bedingungen. So ist beispielsweise bekannt, dass Adenosin die Thrombozyten-Aggregation hemmt und die Durchblutung der Herzkranzgefäße steigert. Weiterhin wirkt es auf den Blutdruck, die Herzfrequenz, auf die Ausschüttung von Neurotransmittern und auf die Lymphozyten-Differenzierung. In Adipozyten ist Adenosin in der Lage, die Lipolyse zu hemmen und somit die Konzentration an freien Fettsäuren und Triglyzeriden im Blut zu senken.

Diese Wirkungen von Adenosin zielen darauf ab, das Sauerstoffangebot der betroffenen Organe zu erhöhen bzw. den Stoffwechsel dieser Organe zu drosseln, um damit unter ischämischen oder hypoxischen Bedingungen eine Anpassung des Organstoffwechsels an die Organdurchblutung zu erreichen.

Die Wirkung von Adenosin wird über spezifische Rezeptoren vermittelt. Bekannt sind bisher die Subtypen A1, A2a, A2b und A3. Als "Adenosinrezeptor-selektive Liganden" werden erfindungsgemäß solche Substanzen bezeichnet, die selektiv an einen oder mehrere Subtypen der Adenosinrezeptoren binden und dabei entweder die Wirkung des Adenosin nachahmen (Adenosin-Agonisten) oder dessen Wirkung blockieren (Adenosin-Antagonisten) können.

Die Wirkungen dieser Adenosin-Rezeptoren werden intrazellulär durch den Botenstoff cAMP vermittelt. Im Falle der Bindung von Adenosin an die A2a- oder A2b-Rezeptoren kommt es über eine Aktivierung der membranständigen Adenylatzyklase zu einem Anstieg des intrazellulären cAMP, während die Bindung des Adenosin an die A1- oder A3-Rezeptoren über eine Hemmung der Adenylatzyklase eine Abnahme des intrazellulären cAMP-Gehalts bewirkt.

Im Herz-Kreislaufsystem sind die Hauptwirkungen der Aktivierung von Adenosin-Rezeptoren: Bradykardie, negative Inotropie und Protektion des Herzens vor Ischämie ("preconditioning") über A1-Rezeptoren, Dilation der Gefäße über A2a- und A2b-Rezeptoren sowie Inhibition der Fibroblasten und Glattmuskelzellproliferation über A2b-Rezeptoren.

Im Falle von A1-Agonisten (Kopplung bevorzugt über Gᵢ-Proteine) wird dabei eine Abnahme des intrazellulären cAMP-Gehaltes beobachtet (bevorzugt nach direkter Vorstimulation der Adenylatzyklase durch Forskolin). Entsprechend führen A2a- und A2b-Agonisten (Kopplung bevorzugt über Gₛ-Proteine) zu einer Zunahme und A2a- und A2b-Antagonisten zu einer Abnahme im cAMP-Gehalt der Zellen. Im Falle der A2-Rezeptoren ist eine direkte Vorstimulation der Adenylatzyklase durch Forskolin nicht hilfreich.

Die Aktivierung von A1-Rezeptoren durch spezifische A1-Agonisten führt beim Menschen zu einer frequenzabhängigen Senkung der Herzfrequenz, ohne einen Einfluss auf den Blutdruck zu haben. Selektive A1-Agonisten könnten somit unter anderem für die Behandlung von Angina pectoris und Vorhofflimmem geeignet sein.

Die kardioprotektive Wirkung der A1-Rezeptoren im Herzen kann unter anderem durch die Aktivierung dieser A1-Rezeptoren durch spezifische A1-Agonisten für die Behandlung und Organprotektion bei akutem Myokardinfarkt, akutem Koronarsyndrom, Herzinsuffizienz, Bypass Operationen, Herzkatheteruntersuchungen und Organtransplantationen genutzt werden.

Die Aktivierung von A2b-Rezeptoren durch Adenosin oder spezifische A2b-Agonisten führt über die Erweiterung von Gefäßen zu einer Blutdrucksenkung. Die Blutdrucksenkung ist von einem reflektorischen Herzfrequenzanstieg begleitet. Der Herafrequenzanstieg kann durch die Aktivierung von A1-Rezeptoren durch spezifische A1-Agonisten reduziert werden.

Die kombinierte Wirkung von selektiven A1/A2b-Agonisten auf das Gefäßsystem und die Herzfrequenz resultiert somit in einer systemischen Blutdrucksenkung ohne relevanten Herzfrequenzanstieg. Mit einem solchen pharmakologischen Profil könnten duale A1/A2b-Agonisten zur Behandlung z.B. der Hypertonie beim Menschen eingesetzt werden.

Die Hemmung von A1-Rezeptoren durch spezifische A1-Antagonisten wirkt beim Menschen urikosurisch, natriuretisch sowie Kalium sparend diuretisch ohne Beeinflussung der glomerulären Filtrationsrate und somit nierenprotektiv. Selektive A1-Antagonisten können somit unter anderem zur Behandlung von akut dekompensierter Herzinsuffizienz und chronischer Herzinsuffizienz geeignet sein. Desweiteren können sie zur Nierenprotektion bei Nephropathie und anderen Nierenerkrankungen eingesetzt werden.

In Adipozyten bewirkt die Aktivierung von A1- und A2b-Rezeptoren eine Inhibition der Lipolyse. Die kombinierte Wirkung von A1/A2b-Agonisten auf den Lipidstoffwechsel führt somit zu einer Senkung von freien Fettsäuren und Triglyzeriden. Eine Senkung der Lipide wiederum führt bei Patienten mit Metabolischem Syndrom und bei Diabetikern zur Verringerung der Insulinresistenz und zur Verbesserung der Symptomatik.

Die zuvor genannte Rezeptor-Selektivität lässt sich bestimmen durch die Wirkung der Substanzen an Zelllinien, die nach stabiler Transfektion mit der entsprechenden cDNA die jeweiligen Rezeptorsubtypen exprimieren (siehe hierzu die Druckschrift M. E. Olah, H. Ren, J. Ostrowski, K. A. Jacobson, G. L. Stiles, "Cloning, expression, and characterization of the unique bovine A1 adenosine receptor. Studies on the ligand binding site by site-directed mutagenesis", J. Biol. Chem. 267 (1992), Seiten 10764-10770).

Die Wirkung der Substanzen an solchen Zelllinien lässt sich erfassen durch biochemische Messung des intrazellulären Botenstoffes cAMP (siehe hierzu die Druckschrift K. N. Klotz, J. Hessling, J. Hegler, C. Owman, B. Kull, B. B. Fredholm, M. J. Lohse, "Comparative pharmacology of human adenosine receptor subtypes - characterization of stably transfected receptors in CHO cells", Naunyn Schmiedebergs Arch. Pharmacol. 357 (1998), Seiten 1-9).

Bei den aus dem Stand der Technik bekannten, als "Adenosinrezeptor-spezifisch" geltenden Liganden handelt es sich überwiegend um Derivate auf Basis des natürlichen Adenosins [S.-A. Poulsen und R. J. Quinn, "Adenosine receptors: New opportunities for future drugs", Bioorganic and Medicinal Chemistry 6 (1998), Seiten 619-641]. Diese aus dem Stand der Technik bekannten Adenosin-Liganden haben jedoch meistens den Nachteil, dass sie nicht wirklich rezeptorspezifisch wirken, schwächer wirksam sind als das natürliche Adenosin oder nach oraler Applikation nur sehr schwach wirksam sind. Deshalb werden sie überwiegend nur für experimentelle Zwecke verwendet. In klinischer Entwicklung befindliche Verbindungen dieser Art eignen sich bislang nur zur intravenösen Applikation.

Die Synthese verschiedener Tetrahydrochinolin-Derivate wird in Synthesis 2006, 14: 2357-2370, Chemistry of Heterocyclic Compounds 1997, 33 (10): 1203-1208 und Phosphorus, Sulfur and Silicon 1991, 57: 293-301 beschrieben. 6,7-Dihydro-5H-cyclopent[b]pyridine sind in Ukrainskii Khimicheskii Zhournal (Russian Edition) 2006, 72 (1-2): 116-120 als Syntheseintermediate beschrieben. WO 2004/014372 offenbart heteroarylisch annelierte Cycloalkenylamine als eNO-Synthase Stimulatoren zur Behandlung kardiovaskulärer Erkrankungen. Pyridinylpyrimidone und Chinazolinone werden in WO 02/48115 zur Behandlung parasitärer Erkrankungen beschrieben. EP 0 608 565 beansprucht verschiedenartig substituierte Pyrido[2,3-d]pyrimidine als Endothelin Rezeptor Antagonisten zur Behandlung von unter anderem akuter Niereninsuffizienz, Bluthochdurck und Myokardinfarkt. In EP 0 537 463 werden substituierte Pyrido[2,3-d]pyrimidine als Herbizide beschrieben. Verschiedenartig annelierte Heterocyclen werden in US 2007/0066630 als Nikotinsäurerezeptor-Agonisten zur Behandlung des metabolischen Syndroms, von Dyslipidemie, kardiovaskulären Erkrankungen sowie Erkrankungen des peripheren und zentralen Nervensystems offenbart.

Pyridine, die in 5,6-Position mit dem Fragment -C(O)-N(R⁶)-C(O)-N(R⁶) - anneliert sind ("Pyrido[2,3-d]pyrimidindione") und deren therapeutische Verwendbarkeit als Adenosine Rezeptor Antagonisten gegen Krankheiten wie Myokardinfarkt, und einigen Henkreislauferkrankungen ist aus Bioorg. Med. Chem. 2006, 14, 2837-2849 bekannt.

In WO 2001/025210 werden substituierte 2-Heteroalkylthio-3,5-dicyano-4-aryl-6-Aminopyridine als Adenosin Rezeptor Liganden zur therapeutischen Anwendung bei Krankheiten wie beispielsweise kardiovaskulären Erkrankungen offenbart.

US 2006/014764 A1 betrifft Pyrido[2,3-d]pyrimidindione, denen das 7-"-X-CH 2-R" Fragment fehlt, und deren Verwendung als Dipeptidyl Peptidase Inhibitoren.

Aufgabe der vorliegenden Erfindung ist die Bereitstellung neuer Verbindungen, die als selektive Liganden des Adenosin A1- und/oder des Adenosin A2b-Rezeptors wirken und als solche zur Behandlung und/oder Prävention von Krankheiten, insbesondere zur Behandlung und/oder Prävention von kardiovaskulären Erkrankungen.

Gegenstand der vorliegenden Erfindung sind Verbindungen der Formel (I) 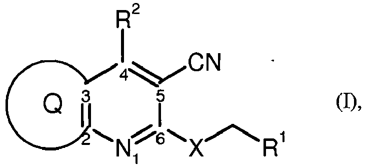 in welcher
der Ring Q für eine Gruppe der Formel 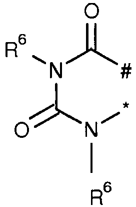 steht, wobei
- *: die Anknüpfstelle an das C2-Atom bedeutet,
- #: die Anknüpfstelle an das C3-Atom bedeutet,
- R⁶: für jeweils Wasserstoff, (C₁-C₄)-Alkyl oder Allyl steht, worin (C₁-C₄)-Alkyl mit einem Substituenten ausgewählt aus der Gruppe Hydroxycarbonyl, (C₁-C₄)-Alkoxycarbonyl und Amino substituiert sein kann,

- X: für S oder O steht,
- R¹: für (C₆-C₁₀)-Aryl oder 5- bis 10-gliedriges Heteroaryl steht,
wobei (C₆-C₁₀)-Aryl und 5- bis 10-gliedriges Heteroaryl mit 1 oder 2 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Hydroxycarbonyl, (C₁-C₆)-Alkoxycarbonyl, Aminocarbonyl, Mono-(C₁-C₆)-alkylaminocarbonyl, Di-(C₁-C₆)-alkylaminocarbonyl, Pyrrolidino, Piperidino, Morpholino, Piperazino und N'-(C₁-C₄)-Alkylpiperazino, Phenyl und 5- oder 6-gliedriges Heteroaryl, substituiert sein können,
worin Phenyl und 5- oder 6-gliedriges Heteroaryl mit 1 bis 3 Substituenten unabhängig voneinander ausgewählt aus der Gruppe Halogen, Nitro, Cyano, (C₁-C₆)-Alkyl, Difluormethyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl substituiert sein können,
- R²: für (C₅-C₆)-Cycloalkyl, 5- oder 6-gliedriges Heterocyclyl, Phenyl oder 5- oder 6-gliedriges Heteroaryl steht,
wobei (C₅-C₆)-Cycloalkyl mit 1 oder 2 Substituenten unabhängig voneinander ausgewählt aus der Gruppe (C₁-C₆)-Alkyl, Hydroxy, Oxo, (C₁-C₆)-Alkoxy, Amino, Mono-(C₁-C₆)-alkylanüno und Di-(C₁-C₆)-alkylamino substituiert sein kann,
worin (C₁-C₆)-Alkyl und (C₁-C₆)-Alkoxy mit 1 oder 2 Substituenten unabhängig voneinander ausgewählt aus der Gruppe Hydroxy, (C₁-C₄)-Alkoxy und (C₃-C₇)-Cycloalkyl substituiert sein können,
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann, und
wobei 5- oder 6-gliedriges Heterocyclyl mit 1 bis 3 Substituenten unabhängig voneinander ausgewählt aus der Gruppe Oxo, Thioxo, Hydroxy, (C₁-C₆)-Alkyl, (C₁-C₆)-Alkoxy, (C₁-C₆)-Alkylcarbonyl, Amino, Mono-(C₁-C₆)-alkylamino, Di-(C₁-C₆)-alkylamino und (C₃-C₇)-Cycloalkyl substituiert sein kann,
worin (C₁-C₆)-Alkyl mit 1 bis 3 Substituenten unabhängig voneinander ausgewählt aus der Gruppe Fluor, Oxo, Hydroxy, Trifluormethyl, (C₁-C₄)-Alkoxy, (C₁-C₄)-Alkylcarbonyloxy, Amino, Mono-(C₁-C₄)-alkylamino, Di-(C₁-C₄)-alkylamino und (C₃-C₇)-Cycloalkyl substituiert sein kann,
   worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
   und
worin (C₁-C₆)-Alkylcarbonyl mit einem Substituenten ausgewählt aus der Gruppe Hydroxy und (C₁-C₄)-Alkoxy substituiert sein kann,
   und
worin (C₃-C₇)-Cycloalkyl mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
wobei Phenyl und 5- oder 6-gliedriges Heteroaryl mit 1 bis 3 Substituenten unabhängig voneinander ausgewählt aus der Gruppe Halogen, Cyano, Hydroxy, (C₁-C₆)-Alkyl, (C₁-C₆)-Alkoxy, (C₃-C₇)-Cycloalkoxy und -NR^{A}R^{B} substituiert sein können,
worin (C₁-C₆)-Alkyl mit 1 bis 3 Substituenten Fluor substituiert sein kann,
und
worin (C₁-C₆)-Alkoxy mit 1 bis 3 Substituenten unabhängig voneinander ausgewählt aus der Gruppe Fluor, Trifluormethyl, (C₃-C₇)-Cycloalkyl, Oxo, Hydroxy, (C₁-C₄)-Alkoxy, Hydroxycarbonyl, Amino, Mono-(C₁-C₄)-alkylamino und Di-(C₁-C₄)-alkylamino substituiert sein kann,
und
worin (C₃-C₇)-Cycloalkoxy mit 1 oder 2 Substituenten unabhängig voneinander ausgewählt aus der Gruppe (C₁-C₄)-Alkyl, Hydroxy, Oxo und (C₁-C₄)-Alkoxy substituiert sein können,
und
worin
   R^{A} für Wasserstoff oder (C₁-C₆)-Alkyl steht,
   worin (C₁-C₆)-Alkyl seinerseits mit einem Substituenten ausgewählt aus der Gruppe Hydroxy und (C₁-C₄)-Alkoxy substituiert sein kann,
   R^{B} für Wasserstoff, (C₁-C₆)-Alkyl, (C₃-C₇)-Cycloalkyl, (C₁-C₄)-Alkylsulfonyl oder (C₃-C₇)-Cycloalkylsulfonyl steht,
   worin (C₁-C₆)-Alkyl seinerseits mit 1 oder 2 Substituenten unabhängig voneinander ausgewählt aus der Gruppe (C₃-C₇)-Cycloalkyl, Oxo, Hydroxy, (C₁-C₄)-Alkoxy, Hydroxycarbonyl, Amino, Mono-(C₁-C₄)-alkylamino und Di-(C₁-C₄)-alkylamino substituiert sein kann, und
   worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
oder
worin zwei benachbarte Substituenten am Phenyl zusammen mit den Kohlenstoffatomen, an die sie gebunden sind, ein 1,3-Dioxolan oder 2,2-Difluor-1,3-dioxolan bilden können,
sowie ihre N-Oxide, Salze, Solvate, Salze der *N*-Oxide und Solvate der *N*-Oxide.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Anmioniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit 1 bis 6 bzw. 1 bis 4 Kohlenstoffatomen. Bevorzugt ist ein linearer oder verzweigter Alkylrest mit 1 bis 4 Kohlenstoffatomen. Beispielhaft und vorzugsweise seien genannt: Methyl, Ethyl, n-Propyl, Isopropyl, n-Butyl, iso-Butyl, sec.-Butyl, tert.-Butyl, 1-Ethylpropyl, n-Pentyl und n-Hexyl.
Cycloalkyl steht im Rahmen der Erfindung für einen monocyclischen, gesättigten Carbocyclus mit 3 bis 7 bzw. 5 bis 6 Ring-Kohlenstoffatomen. Beispielhaft und vorzugsweise seien genannt: Cyclopropyl, Cyclobutyl, Cyclopentyl, Cyclohexyl und Cycloheptyl.
Alkylcarbonyl steht im Rahmen der Erfindung für einen linearen oder verzweigten Alkylrest mit 1 bis 6 bzw. 1 bis 4 Kohlenstoffatomen und einer in 1-Position angebundenen Carbonylgruppe. Beispielhaft und vorzugsweise seien genannt: Methylcarbonyl, Ethylcarbonyl, n-Propylcarbonyl, iso-Propylcarbonyl, n-Butylcarbonyl, iso-Butylcarbonyl und tert.-Butylcarbonyl.
Alkylcarbonyloxy steht im Rahmen der Erfindung für einen linearen oder verzweigten Alkylrest mit 1 bis 4 Kohlenstoffatomen und einer in 1-Position angebundenen Carbonylgruppe, der über ein Sauerstoffatom verknüpft ist. Beispielhaft und vorzugsweise seien genannt: Methylcarbonyloxy, Ethylcarbonyloxy, n-Propylcarbonyloxy, Isopropylcarbonyloxy und tert.-Butylcarbonyloxy.
Alkoxy steht im Rahmen der Erfindung für einen linearen oder verzweigten Alkoxyrest mit 1 bis 6 bzw. 1 bis 4 bzw. 2 bis 4 Kohlenstoffatomen. Bevorzugt ist ein linearer oder verzweigter Alkoxyrest mit 1 bis 4 bzw. 2 bis 4 Kohlenstoffatomen. Beispielhaft und vorzugsweise seien genannt: Methoxy, Ethoxy, n-Propoxy, Isopropoxy, n-Butoxy, tert.-Butoxy, n-Pentoxy und n-Hexoxy.
Cycloalkoxy steht im Rahmen der Erfindung für einen monocyclischen, gesättigten Alkoxyrest mit 3 bis 7 Kohlenstoffatomen. Beispielhaft und vorzugsweise seien genannt: Cyclopropyloxy, Cyclobutyloxy, Cyclopentyloxy, Cyclohexyloxy und Cycloheptyloxy.
Alkoxycarbonyl stehen im Rahmen der Erfindung für einen linearen oder verzweigten Alkoxyrest mit 1 bis 6 bzw. 1 bis 4 Kohlenstoffatomen und einer am Sauerstoff angebundenen Carbonylgruppe. Bevorzugt ist ein linearer oder verzweigter Alkoxycarbonylrest mit 1 bis 4 Kohlenstoffatomen in der Alkoxy-Gruppe. Beispielhaft und vorzugsweise seien genannt: Methoxycarbonyl, Ethoxycarbonyl, n-Propoxycarbonyl, Isopropoxycarbonyl und tert.-Butoxycarbonyl.
Mono-alkylamino steht im Rahmen der Erfindung für eine Amino-Gruppe mit einem linearen oder verzweigten Alkylsubstituenten, der 1 bis 6 bzw. 1 bis 4 bzw. 2 bis 4 Kohlenstoffatome aufweist. Bevorzugt ist ein linearer oder verzweigter Monoalkylamino-Rest mit 1 bis 4 bzw. 2 bis 4 Kohlenstoffatomen. Beispielhaft und vorzugsweise seien genannt: Methylamino, Ethylamino, n-Propylamino, Isopropylamino, n-Butylamino, tert.-Butylamino, n-Pentylamino und n-Hexylamino.
Di-alkylamino steht im Rahmen der Erfindung für eine Amino-Gruppe mit zwei gleichen oder verschiedenen linearen oder verzweigten Alkylsubstituenten, die jeweils 1 bis 6 bzw. 1 bis 4 Kohlenstoffatome aufweisen. Bevorzugt sind lineare oder verzweigte Dialkylamino-Reste mit jeweils 1 bis 4 Kohlenstoffatomen. Beispielhaft und vorzugsweise seien genannt: *N,N*-Dimethylamino, *N,N*-Diethylamino, *N*-Ethyl-N-methylamino, *N*-Methyl-*N*-n-propylamino, *N*-Isopropyl-*N*-n-propylamino, *N,N-*Diisopropylamino, *N*-n-Butyl-*N*-methylamino, *N*-tert.-Butyl-*N*-methylamino, *N*-Ethyl-*N*-n-pentylamino und *N*-n-Hexyl-*N*-methylamino.
Mono-alkylaminocarbonyl steht im Rahmen der Erfindung für eine Amino-Gruppe, die über eine Carbonylgruppe verknüpft ist und die einen linearen oder verzweigten Alkylsubstituenten mit 1 bis 6 bzw. 1 bis 4 Kohlenstoffatomen aufweist. Bevorzugt ist ein Mono-alkylaminocarbonyl-Rest mit 1 bis 4 Kohlenstoffatomen in der Alkylgruppe. Beispielhaft und vorzugsweise seien genannt: Methylaminocarbonyl, Ethylaminocarbonyl, *n*-Propylaminocarbonyl, Isopropylaminocarbonyl, *n*-Butylaminocarbonyl und *tert*.-Butylaminocarbonyl.
Di-alkylaminocarbonyl steht im Rahmen der Erfindung für eine Amino-Gruppe, die über eine Carbonylgruppe verknüpft ist und die zwei gleiche oder verschiedene lineare oder verzweigte Alkylsubstituenten mit jeweils 1 bis 6 bzw. 1 bis 4 Kohlenstoffatomen aufweist. Bevorzugt ist ein Dialkylaminocarbonyl-Rest mit jeweils 1 bis 4 Kohlenstoffatomen pro Alkylgruppe. Beispielhaft und vorzugsweise seien genannt: *N,N*-Dimethylaminocarbonyl, *N,N*-Diethylaminocarbonyl, *N*-Ethyl-*N*-methylaminocarbonyl, *N*-Methyl-*N*-*n*-propylaminocarbonyl, *N*-*n*-Butyl-N-methylaminocarbonyl und *N*-tert.-Butyl-*N*-methylanlinocarbonyl.
Alkylimino steht im Rahmen der Erfindung für eine Imino-Gruppe mit einem linearen oder verzweigten Alkylsubstituenten, der 1 bis 4 Kohlenstoffatome aufweist. Beispielhaft und vorzugsweise seien genannt: MethylAmino, Ethylimino, n-Propylimino, Isopropylimino, n-Butylimino und tert.-Butylimino.
Alkoxyimino steht im Rahmen der Erfindung für eine Imino-Gruppe mit einem linearen oder verzweigten Alkoxysubstituenten, der 1 bis 4 Kohlenstoffatome aufweist. Beispielhaft und vorzugsweise seien genannt: Methoxyimino, Ethoxyimino, n-Propoxyimino, Isopropoxyimino, n-Butoxyimino und tert.-Butoxyimino.
Alkylsulfonyl steht in Rahmen der Erfindung für einen linearen oder verzweigten Alkylrest mit 1 bis 4 Kohlenstoffatomen, der über eine Sulfongruppe gebunden ist. Beispielhaft und vorzugsweise seinen genannt: Methylsulfonyl, Ethylsulfonyl, n-Propylsulfonyl, iso-Propylsulfonyl, n-Butylsulfonyl und tert.-Butylsulfonyl.
Cycloalkylsulfonyl steht in Rahmen der Erfindung für einen monocyclischen, gesättigten Alkylrest mit 3 bis 7 Kohlenstoffatomen, der über eine Sulfongruppe gebunden ist. Beispielhaft und vorzugsweise seinen genannt: Cyclopropylsulfonyl, Cyclobutylsulfonyl, Cyclopentylsulfonyl, Cyclohexylsulfonyl und Cycloheptylsulfonyl.
Heterocyclyl steht im Rahmen der Erfindung für einen gesättigten Heterocyclus mit insgesamt 5 oder 6 Ringatomen, der ein oder zwei Ring-Heteroatome aus der Reihe N, O und/oder S enthält und über ein Ring-Köhlenstoffatom oder gegebenenfalls ein Ring-Stickstoffatom verknüpft ist. Beispielhaft seien genannt: Pyrrolidinyl, Pyrazolidinyl, Tetrahydrofuranyl, Piperidinyl, Piperazinyl, Tetrahydropyranyl, Morpholinyl, Thiomorpholinyl, Hexahydroazepinyl und Hexahydro-1,4-diazepinyl. Bevorzugt sind Pyrrolidinyl, Tetrahydrofuranyl, Piperidinyl, Piperazinyl, Tetrahydropyranyl und Morpholinyl.
(C₆-C₁₀)-Aryl steht im Rahmen der Erfindung für einen aromatischen Carbocyclus mit 6 oder 10 RingKohlenstoffatomen. Bevorzugte Arylreste sind Phenyl und Naphthyl.
Heteroaryl steht im Rahmen der Erfindung für einen mono- oder gegebenenfalls bicyclischen aromatischen Heterocyclus (Heteroaromaten) mit insgesamt 5 bis 10 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl. Bevorzugt sind monocyclische 5- oder 6-gliedriges Heteroaryl-Reste mit bis zu drei Ring-Heteroatomen aus der Reihe N, O und/oder S wie beispielsweise Furyl, Thienyl, Thiazolyl, Oxazolyl, Isothiazolyl, Isoxazolyl, Pyrazolyl, Imidazolyl, Triazolyl, Oxadiazolyl, Thiadiazolyl, Pyridyl, Pyrimidinyl, Pyridazinyl, Pyrazinyl, Triazinyl.
Halogen schließt im Rahmen der Erfindung Fluor, Chlor, Brom und Iod ein. Bevorzugt sind Chlor oder Fluor.

In den Formeln der Gruppe, die für Q stehen kann, steht der Endpunkt der Linie, neben der jeweils ein * bzw. # steht, nicht für ein Kohlenstoffatom beziehungsweise eine CH₂-Gruppe sondern ist Bestandteil der Bindung zu dem Atom, an das Q gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei gleichen oder verschiedenen Substituenten.

Bevorzugt sind im Rahmen der vorliegenden Erfindung Verbindungen der Formel (I), in welcher der Ring Q für eine Gruppe der Formel 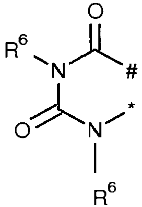 steht, wobei
- *: die Anknüpfstelle an das C2-Atom bedeutet,
- #: die Anknüpfstelle an das C3-Atom bedeutet,
- R⁶: für jeweils Wasserstoff oder Methyl steht,
- X: für S oder O steht,
- R¹: für Phenyl oder 5- oder 6-gliedriges Heteroaryl steht,
wobei Phenyl oder 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, Hydroxy, (C₁-C₄)-Alkoxy, Amino, Hydroxycarbonyl, (C₁-C₄)-Alkoxycarbonyl, Aminocarbonyl, Phenyl und 5- oder 6-gliedriges Heteroaryl substituiert sind,
worin Phenyl und 5- oder 6-gliedriges Heteroaryl mit 1 bis 3 Substituenten unabhängig voneinander ausgewählt aus der Gruppe Fluor, Chlor, Nitro, Cyano, (C₁-C₄)-Alkyl, Difluormethyl, Trifluormethyl, Hydroxy, (C₁-C₄)-Alkoxy, Difluormethoxy, Trifluormethoxy, Amino, Hydroxycarbonyl und (C₁-C₄)-Alkoxycarbonyl substituiert sein können,
- R²: für Cyclohexyl, Tetrahydropyranyl, Piperidinyl, Piperazinyl, Morpholinyl, Phenyl, Pyrazolyl, Imidazolyl, Oxazolyl, Thiazolyl oder Pyridyl steht,
wobei Cyclohexyl mit einem Substituenten ausgewählt aus der Gruppe Hydroxy und (C₁-C₄)-Alkoxy substituiert sein kann,
worin (C₂-C₄)-Alkoxy mit 1 oder 2 Substituenten unabhängig voneinander ausgewählt aus der Gruppe Hydroxy und Methoxy substituiert sein kann,
und
wobei Piperidinyl, Piperazinyl und Morpholinyl mit einem Substituenten ausgewählt aus der Gruppe (C₁-C₄)-Alkyl, Hydroxy, (C₁-C₄)-Alkoxy und (C₁-C₄)-Alkylcarbonyl substituiert sein können,
worin (C₁-C₄)-Alkyl mit 1 oder 2 Substituenten unabhängig voneinander ausgewählt aus der Gruppe Hydroxy, Methoxy, Ethoxy, Methylcarbonyloxy und Ethylcarbonyloxy substituiert sein kann,
und
worin (C₁-C₄)-Alkylcarbonyl mit einem Substituenten ausgewählt aus der Gruppe Hydroxy, Methoxy und Ethoxy substituiert sein kann,
und
wobei Phenyl und Pyridyl mit 1 bis 3 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können,
worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
und
wobei Pyrazolyl, Imidazolyl, Oxazolyl und Thiazolyl mit 1 oder 2 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können,
worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
sowie ihre Salze, Solvate und Solvate der Salze.

Besonders bevorzugt sind im Rahmen der vorliegenden Erfindung Verbindungen der Formel (I), in welcher der Ring Q für eine Gruppe der Formel 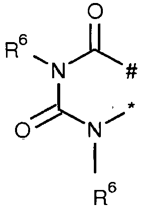 steht, wobei
- *: die Anknüpfstelle an das C2-Atom bedeutet,
- #: die Anknüpfstelle an das C3-Atom bedeutet,
- R⁶: für jeweils Wasserstoff oder Methyl steht,
- X: für S oder O steht,
- R¹: für Phenyl oder 5- oder 6-gliedriges Heteroaryl steht,
wobei Phenyl oder 5- oder 6-gliedriges Heteroaryl mit 1 oder 2 Substituenten unabhängig voneinander ausgewählt aus der Gruppe Fluor, Chlor, Cyano, Methyl, Ethyl, Trifluormethyl, Hydroxy, Methoxy, Ethoxy, Amino, Hydroxycarbonyl, Methoxycarbonyl, Ethoxycarbonyl, Aminocarbonyl, Phenyl und 5-oder 6-gliedriges Heteroaryl substituiert sind,
worin Phenyl und 5- oder 6-gliedriges Heteroaryl mit 1 bis 3 Substituenten unabhängig voneinander ausgewählt aus der Gruppe Fluor, Chlor, Methyl, Ethyl, Difluonnethyl, Trifluormethyl, Hydroxy, Methoxy, Ethoxy, Amino, Hydroxycarbonyl, Methoxycarbonyl und Ethoxycarbonyl substituiert sein können,
- R²: für Phenyl, Pyrazolyl oder Pyridyl steht,
wobei Phenyl und Pyridyl mit 1 oder 2 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können,
worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
und
wobei Pyrazolyl mit einem Substituenten ausgewählt aus der Gruppe Fluor, Chlor, Cyano, Hydroxy, (C₁-C₄)-Alkyl und (C₁-C₄)-Alkoxy substituiert sein kann,
worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
sowie ihre Salze, Solvate und Solvate der Salze.

Ganz besonders bevorzugt sind im Rahmen der vorliegenden Erfindung Verbindungen der Formel (I), in welcher der Ring Q für eine Gruppe der Formel 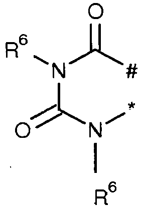 steht, worin
- *: die Anknüpfstelle an das C2-Atom bedeutet,
- #: die Anknüpfstelle an das C3-Atom bedeutet,
- R⁶: für Wasserstoff steht,
- X: für S oder O steht,
- R¹: für Thiazolyl oder Oxazolyl steht,
wobei Thiazolyl und Oxazolyl mit einem Substituenten Phenyl substituiert sind,
worin Phenyl mit einem Substituenten ausgewählt aus der Gruppe Fluor, Chlor, Cyano, Methyl, Methoxy, Hydroxycarbonyl und Methoxycarbonyl substituiert sein kann,
und
wobei Thiazolyl und Oxazolyl mit einem Substituenten ausgewählt aus der Gruppe Fluor, Chlor, Cyano, Methyl, Ethyl, Methoxy, Amino, Hydroxycarbonyl und Methoxycarbonyl substituiert sein können,
- R²: für eine Gruppe der Formel 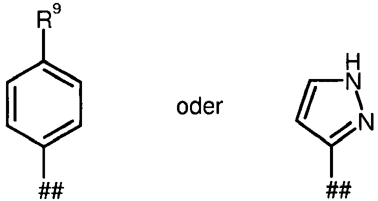 steht, wobei
## die Anknüpfstelle an den Bicyclus bedeutet, worin
R⁹ für Wasserstoff oder (C₁-C₄)-Alkoxy steht, worin (C₂-C₄)-Alkoxy mit 1 oder 2 Substituenten Hydroxy substituiert sein kann,
sowie ihre Salze, Solvate und Solvate der Salze.

Bevorzugt sind im Rahmen der vorliegenden Erfindung auch Verbindungen der Formel (I), in welcher
- R¹: für Thiazolyl oder Oxazolyl steht,
wobei Thiazolyl und Oxazolyl mit einem Substituenten Phenyl substituiert sind,
worin Phenyl mit einem Substituenten ausgewählt aus der Gruppe Fluor, Chlor, Cyano, Methyl, Methoxy, Hydroxycarbonyl und Methoxycarbonyl substituiert sein kann,
und
wobei Thiazolyl und Oxazolyl mit einem Substituenten ausgewählt aus der Gruppe Fluor, Chlor, Cyano, Methyl, Ethyl, Methoxy, Amino, Hydroxycarbonyl und Methoxycarbonyl substituiert sein können.

Bevorzugt sind im Rahmen der vorliegenden Erfindung auch Verbindungen der Formel (I), in welcher substituiert sein können,
- R²: für eine Gruppe der Formel 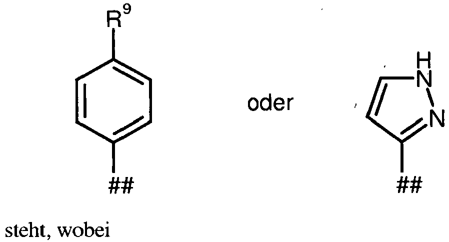
- ##: die Anknüpfstelle an den Bicyclus bedeutet,
worin
- R⁹: für Wasserstoff oder (C₁-C₄)-Alkoxy steht, worin (C₂-C₄)-Alkoxy mit 1 oder 2 Substituenten Hydroxy substituiert sein kann.

Weiterer Gegenstand der vorliegenden Erfindung ist ein Verfahren zur Herstellung der erfindungsgemäßen Verbindungen der Formel (I), dadurch gekennzeichnet, dass man
[C] eine Verbindung der Formel (V) 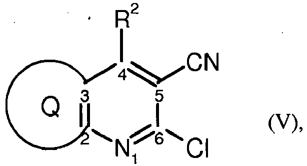 in welcher R² die oben angegebene Bedeutung hat und
   der Ring Q für eine Gruppe der Formel 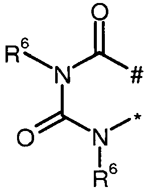 steht, wobei
   - *: jeweils die Anknüpfstelle an das C2-Atom bedeutet,
   - #: jeweils die Anknüpfstelle an das C3-Atom bedeutet,
   - R⁶: für jeweils Wasserstoff, (C₁-C₄)-Alkyl oder Allyl steht, worin (C₁-C₄)-Alkyl mit einem Substituenten ausgewählt aus der Gruppe Hydroxycarbonyl, (C₁-C₄)-Alkoxycarbonyl und Amino substituiert sein kann,
   in einem inerten Lösungsmittel zuerst mit einem Alkalisulfid, wie beispielsweise Natriumsulfid, zu einer Verbindung der Formel (VI) 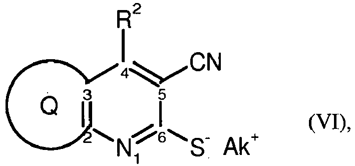 in welcher R² die oben angegebene Bedeutung hat,
   Ak⁺ für ein Alkalisalz, vorzugsweise Natriumsalz, steht
   und
   der Ring Q für eine Gruppe der Formel 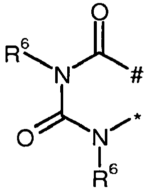 steht, wobei
   - *: jeweils die Anknüpfstelle an das C2-Atom bedeutet,
   - #: jeweils die Anknüpfstelle an das C3-Atom bedeutet,
   - R⁶: für jeweils Wasserstoff, (C₁-C₄)-Alkyl oder Allyl steht, worin (C₁-C₄)-Alkyl mit einem Substituenten ausgewählt aus der Gruppe Hydroxycarbonyl, (C₁-C₄)-Alkoxycarbonyl und Amino substituiert sein kann,
   umsetzt und diese anschliessend in Gegenwart einer geeigneten Base mit der Verbindung der Formel (VII) 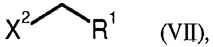 in welcher R¹ die oben angegebene Bedeutung hat und
   - X²: für eine geeignete Abgangsgruppe, vorzugsweise für Halogen, insbesondere Chlor, Brom oder Iod, oder für Mesylat, Tosylat oder Triflat steht,
   zu einer Verbindung der Formel (I-C) 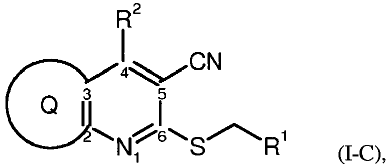 in welcher R¹ und R² jeweils die oben angegebenen Bedeutungen haben,
   der Ring Q für eine Gruppe der Formel 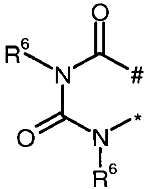 steht, wobei
   - *: jeweils die Anknüpfstelle an das C2-Atom bedeutet,
   - #: jeweils die Anknüpfstelle an das C3-Atom bedeutet,
   - R⁶: für Wasserstoff, (C₁-C₄)-Alkyl oder Allyl steht, worin (C₁-C₄)-Alkyl mit einem Substituenten ausgewählt aus der Gruppe Hydroxycarbonyl, (C₁-C₄)-Alkoxycarbonyl und Amino substituiert sein kann,
   umsetzt,
   oder
[D] eine Verbindung der Formel (V) in einem inerten Lösungsmittel in Gegenwart einer Base mit einer Verbindung der Formel (VIII) 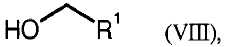 in welcher R¹ die oben angegebene Bedeutung hat
   zu Verbindungen der Formel (I-D) 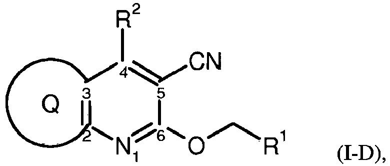 in welcher R¹ und R² jeweils die oben angegebenen Bedeutungen haben,
   und
   der Ring Q für eine Gruppe der Formel 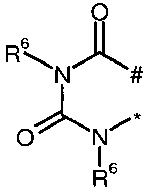 steht, wobei
   - *: jeweils die Anknüpfstelle an das C2-Atom bedeutet,
   - #: jeweils die Anknüpfstelle an das C3-Atom bedeutet,
   - R⁶: für Wasserstoff, (C₁-C₄)-Alkyl oder Allyl steht, worin (C₁-C₄)-Alkyl mit einem Substituenten ausgewählt aus der Gruppe Hydroxycarbonyl, (C₁-C₄)-Alkoxycarbonyl und Amino substituiert sein kann,
   umsetzt,
anschließend die resultierenden Verbindungen der Formeln (I-C) und (I-D) gegebenenfalls mit den entsprechenden (i) Lösungsmitteln und/oder (ii) Basen oder Säuren in ihre Solvate, Salze und/oder Solvate der Salze überführt.

In den Verbindungen der Formeln (V) bzw. in den Resten R² und/oder R⁶ gegebenenfalls vorhandene funktionelle Gruppen - wie insbesondere Amino-, Hydroxy- und Carboxylgruppen - können bei diesem Verfahren, falls zweckmäßig oder erforderlich, auch in temporär geschützter Form vorliegen. Die Einführung und Entfernung solcher Schutzgruppen erfolgt hierbei nach üblichen, dem Fachmann bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]. Bei Vorhandensein mehrerer Schutzgruppen kann die Entfernung gegebenenfalls simultan in einer Eintopf-Reaktion oder in separaten Reaktionsschritten vorgenommen werden.

Weitere erfindungsgemäße Verbindungen können gegebenenfalls ausgehend von den nach obigen Verfahren erhaltenen Verbindungen (I-C) und (I-D) auch hergestellt werden durch Umwandlungen von funktionellen Gruppen einzelner Substituenten, insbesondere den unter R² und Q aufgeführten. Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetallkatalysierte Kupplungsreaktionen, Eliminierungen, Alkylierung, Aminierung, Veresterung, Esterspaltung, Veretherung, Etherspaltung, Bildung von Carbonamiden, sowie Einführung und Entfernung temporärer Schutzgruppen.

Die Verbindungen der Formel (VII) sind kommerziell erhältlich, literaturbekannt oder nach literaturbekannten Methoden herstellbar. So können beispielsweise durch Reaktion von Amiden, Thioamiden bzw. Thioharnstoff-Derivaten mit einem 1,3-Dihalogenaceton substituierte Oxazol- und Thiazol-Derivate der Formel (VII-A) und (VII-B) erhalten werden (siehe Schema 1):

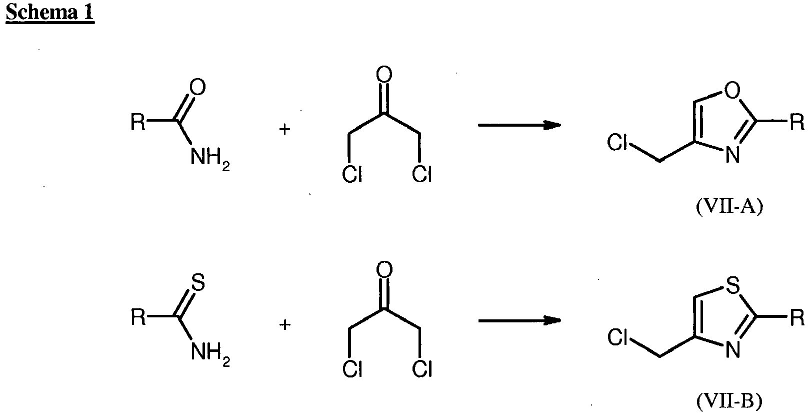

Die Verbindungen der Formel (VIII) sind kommerziell erhältlich, literaturbekannt oder nach literaturbekannten Methoden herstellbar. [vgl. z.B. M. Suzuki et al., J. Org. Chem. 1973, 38, 3571-3575; E.A. Krasnokutskaya et al., Synthesis 2007, 1, 81-84; J. Hassan et al., Chem. Rev. 2002, 102, 1359-1469].

Die Verbindungen der Formel (V) können hergestellt werden, indem man eine Verbindung der Formel (XVII) 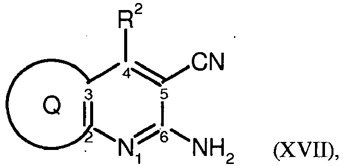 in welcher Q und R² die oben angegebenen Bedeutungen haben,
in einem inerten Lösungsmittel mit Kupfer(II)chlorid und Isopentylnitrit umsetzt.

Die Verbindungen (XVII) sind literaturbekannt oder können in Analogie zu literaturbekannten Verfahren hergestellt werden [vgl. z.B. Assy et al., J. Indian. Chem. Soc. 1996, 73(11), 623-624 und Kambe et al., Synthesis 1980, 5, 366-368]

Inerte Lösungsmittel für die Umsetzung(VI) + (VII) → (I-C) sind beispielsweise Alkohole wie Methanol, Ethanol, n-Propanol, Isopropanol, n-Butanol und tert.-Butanol, Ketone wie Aceton und Methylethylketon, acyclische und cyclische Ether wie Diethylether, Methyl-tert.-butylether, 1,2-Dimethoxyethan, Tetrahydrofuran und Dioxan, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, Trichlormethan und Chlorbenzol, oder andere Lösungsmittel wie Dimethylformamid (DMF), Dimethylsulfoxid (DMSO), N-Methylpyrrolidinon (NMP), Acetonitril oder Pyridin. Wasser ist als Lösungsmittel ebenfalls geeignet. Ebenso ist es möglich, Gemische der zuvor genannten Lösungsmittel einzusetzen. Bevorzugt wird Dimethylformamid als Lösungsmittel verwendet.

Als Base für die Umsetzung (VI) + (VII) → (I-C) eignen sich die üblichen anorganischen oder organischen Basen. Hierzu gehören bevorzugt Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt sind Alkalicarbonate und Alkalihydrogencarbonate.

Die Base kann hierbei in einer Menge von 1 bis 10 Mol, bevorzugt von 1 bis 5 Mol, insbesondere von 1 bis 4 Mol, bezogen auf 1 Mol der Verbindung der Formel (VII), eingesetzt werden.

Die Reaktion erfolgt im Allgemeinen in einem Temperaturbereich von -78°C bis +140°C, bevorzugt im Bereich von -20°C bis +80°C, insbesondere bei 0°C bis +50°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als inerte Lösungsmittel für die Reaktionen (V) + (VIII) → (I-D) eignen sich insbesondere acyclische und cyclische Ether wie Diethylether, Methyl-tert.-butylether, 1,2-Dimethoxyethan, Tetrahydrofuran und Dioxan, Kohlenwasserstoffe wie Benzol, Toluol, Xylol, Hexan und Cyclohexan, oder andere Lösungsmittel wie Dimethylformamid (DMF), Dimethylsulfoxid (DMSO), *N*-Methylpyrrolidinon (NMP) und Pyridin. Ebenso ist es möglich, Gemische dieser Lösungsmittel einzusetzen. Bevorzugt wird 1,2-Dimethoxyethan verwendet.

Als Basen für die Umsetzungen (V) + (VIII) → (I-D) sind insbesondere Alkalialkoholate wie Natrium- oder Kaliummethanolat, Natrium- oder Kaliumethanolat oder Natrium- oder Kalium-tert.-butylat, Amide wie Natriumamid, Lithium-, Natrium- oder Kalium-bis-(trimethylsilyl)amid oder Lithiumdiisopropylamid, oder metallorganische Verbindungen wie Butyllithium oder Phenyllithium geeignet. Bevorzugt wird Kalium-tert.-butylat verwendet.

Die Base wird hierbei in der Regel in einer Menge von 1 bis 1.25 Mol, bevorzugt in äquimolarer Menge, bezogen auf 1 Mol der Verbindung der Formel (VIII), eingesetzt.

Die Reaktionen (V) + (VIII) → (I-D) erfolgen im Allgemeinen in einem Temperaturbereich von -20°C bis +120°C, bevorzugt bei +20°C bis +100°C, gegebenenfalls in der Mikrowelle. Die Umsetzungen können bei normalem, erhöhtem oder erniedrigtem Druck durchgeführt werden (z.B. im Bereich von 0.5 bis 5 bar). Im Allgemeinen arbeitet man bei Normaldruck.

Als Alkalisulfid bei der Umsetzung (V) → (VI) wird vorzugsweise Natriumsulfid in einer Menge von 1 bis 10 Mol, bevorzugt von 1 bis 5 Mol, insbesondere von 1 bis 4 Mol, bezogen auf 1 Mol der Verbindung der Formel (V), eingesetzt.

Als Lösungsmittel für die Umsetzung (V) → (VI) eignen sich alle organischen Lösungsmittel, die unter den Reaktionsbedingungen inert sind. Hierzu gehören bevorzugt Dimethylformamid, *N*-Methylpyrrolidinon, Pyridin und Acetonitril. Ebenso ist es möglich, Gemische der zuvor genannten Lösungsmitteln einzusetzen. Besonders bevorzugt ist Dimethylformamid.

Die Reaktion (V) → (VI) erfolgt im Allgemeinen in einem Temperaturbereich von +20°C bis +140°C, bevorzugt im Bereich von +20°C bis +120°C, insbesondere bei +60°C bis +100°C. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Die Umsetzung (XVII) → (V) erfolgt im Allgemeinen in einem Molverhältnis von 2 bis 12 Mol Kupfer(II)chlorid und 2 bis 12 Mol Isopentylnitrit bezogen auf 1 Mol der Verbindung der Formel (I-A).

Als Lösungsmittel für den Verfahrensschritt (XVII) → (V) eignen sich alle organischen Lösungsmittel, die unter den Reaktionsbedingungen inert sind. Hierzu gehören acyclische und cyclische Ether wie Diethylether und Tetrahydrofuran, Ester wie Essigsäureethylester oder Essigsäurebutylester, Kohlenwasserstoffe wie Benzol, Toluol, Xylol, Hexan und Cyclohexan, chlorierte Kohlenwasserstoffe wie Dichlormethan, 1,2-Dichlorethan und Chlorbenzol, oder andere Lösungsmittel wie Dimethylformamid, Acetonitril oder Pyridin. Ebenso ist es möglich, Gemische der zuvor genannten Lösungsmittel einzusetzen. Bevorzugte Lösungsmittel sind Acetonitril und Dimethylformamid.

Die Reaktion erfolgt im Allgemeinen in einem Temperaturbereich von -78°C bis +180°C, bevorzugt im Bereich von 0°C bis +100°C, insbesondere bei +20°C bis +80°C, gegebenenfalls in der Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Das zuvor beschriebene Verfahren kann durch die folgenden Reaktionsschemata 2 und 3 erläutert werden:

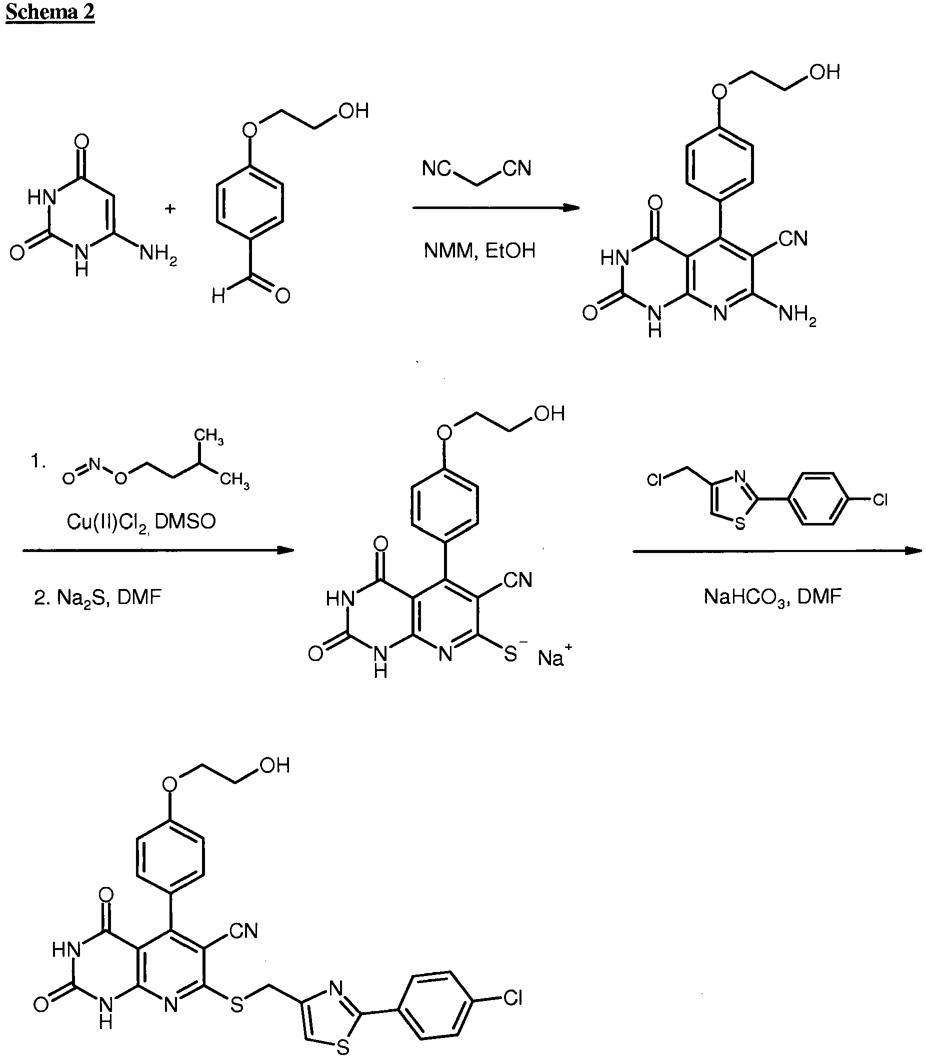

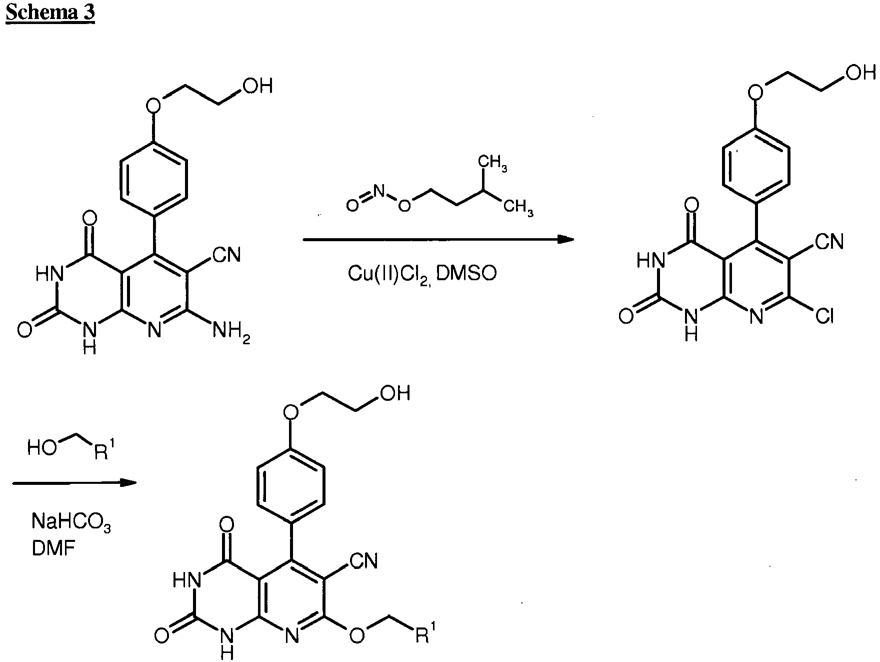

Überraschenderweise zeigen die erfindungsgemäßen Verbindungen ein nicht vorhersehbares, wertvolles pharmakologisches Wirkspektrum und sind daher insbesondere zur Prävention und/oder Behandlung von Erkrankungen geeignet.

Die pharmazeutische Wirksamkeit der erfindungsgemäßen Verbindungen lässt sich durch ihre Wirkung als potente, selektive Liganden an einzelnen oder mehreren Subtypen der Adenosin-Rezeptoren, insbesondere als selektive Liganden an Adenosin A1- und/oder A2b-Rezeptoren erklären. Sie wirken hierbei als selektive A1-Agonisten, selektive A1-Antagonisten oder als selektive duale A1/A2b-Agonisten.

Die erfindungsgemäßen Verbindungen wirken vorwiegend als selektive Adenosin A1-Agonisten.

Als "selektive Liganden an Adenosin A1- und/oder A2b-Rezeptoren" werden im Rahmen der vorliegenden Erfindung solche Adenosin-Rezeptorliganden bezeichnet, bei denen einerseits eine deutliche Wirkung an A1- und/oder A2b-Adenosinrezeptor-Subtypen und andererseits keine oder eine deutliche schwächere Wirkung (Faktor 10 oder höher) an A2a- und A3-Adenosinrezeptor-Subtypen zu beobachten ist, wobei bezüglich der Testmethoden für die Wirk-Selektivität Bezug genommen wird auf die im Abschnitt B-1. beschriebenen Tests.

Die erfindungsgemäßen Verbindungen können in Abhängigkeit von ihrer jeweiligen Struktur als volle, als partielle Adenosinrezeptor-Agonisten oder als Adenosinrezeptor-Antagonisten fungieren. Partielle Adenosinrezeptor-Agonisten sind hierbei definiert als Rezeptorliganden, die eine funktionelle Antwort an Adenosinrezeptoren auslösen, welche geringer ist als bei vollen Agonisten (wie beispielsweise Adenosin selbst). Partielle Agonisten weisen infolgedessen eine geringere Wirksamkeit bezüglich der Rezeptoraktivierung auf als volle Agonisten.

Die Verbindungen der Formel (I) sind allein oder in Kombination mit einem oder mehreren anderen Wirkstoffen zur Herstellung eines Medikaments zur Prävention und/oder Behandlung verschiedener Erkrankungen geeignet, so beispielsweise insbesondere von Hypertonie und anderen Erkrankungen des Herzkreislauf-Systems (kardiovaskulären Erkrankungen), zur Kardioprotektion nach Schädigungen des Herzens sowie von Stoffwechsel- und Nierenerkrankungen.

Im Sinne der vorliegenden Erfindung sind unter Erkrankungen des Herzkreislauf-Systems bzw. kardiovaskulären Erkrankungen neben der Hypertonie beispielsweise die folgenden Erkrankungen zu verstehen: periphere und kardiale Gefäßerkrankungen, koronare Herzerkrankung, koronare Restenose wie z.B. Restenose nach Ballondilatation von peripheren Blutgefäßen, Myokardinfarkt, akutes Koronarsyndrom, stabile und instabile Angina pectoris, Herzinsuffizienz, Tachykardien, Arrhythmien, Vorhof- und Kammerflimmern, periphere Durchblutungsstörungen, erhöhte Spiegel von Fibrinogen und von LDL geringer Dichte, sowie erhöhte Konzentrationen von Plasminogenaktivator-Inhibitor 1 (PAI-1), insbesondere koronare Herzerkrankung, akutes Koronarsyndrom, Angina pectoris, Herzinsuffizienz, Myokardinfarkt, Vorhofflimmern und Hypertonie

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akute dekompensierte Herzinsuffizienz, Rechtsherzinsuffizienz, Linksherzinsuffizienz, Globalinsuffizienz, ischämische Kardiomyopathie, dilatative Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische sowie systolische Herzinsuffizienz.

Weiterhin eignen sich die erfindungsgemäßen Verbindungen auch zur Reduktion des von einem Infarkt betroffenen Myokardbereichs sowie zur Prävention von Sekundärinfarkten.

Des weiteren sind die erfindungsgemäßen Verbindungen zur Prävention und/oder Behandlung von thromboembolischen Erkrankungen, Reperfusionsschäden nach Ischämie, mikro- und makrovaskulären Schädigungen (Vaskulitis), Ödemen, Ischämien wie Myokardinfarkt, Hirnschlag und transitorischen ischämischen Attacken, sowie zur Organprotektion bei Transplantationen, Bypass Operationen, Herzkatheteruntersuchungen und anderen operativen Eingriffen geeignet.

Weiterhin eignen sich die erfindungsgemäßen Verbindungen zur Verwendung bei der Behandlung und/oder Prävention von Nierenerkrankungen, insbesondere von Niereninsuffizienz. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, obstruktive Uropathie, Glomerulonephritis, akute Glomerulonephritis, tubulointerstitielle Erkrankungen, nephropathische Erkrankungen wie primäre und angeborene Nierenerkrankung, Nierenentzündung, durch toxische Substanzen induzierte Nephropathie, diabetische Nephropathie, Pyelonephritis, Nierenzysten und Nephrosklerose, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen in einem Verfahren zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weitere Indikationsgebiete, für die die erfindungsgemäßen Verbindungen eingesetzt werden können, sind beispielsweise die Prävention und/oder Behandlung von Erkrankungen des Urogenitalbereiches, wie z.B. Reizblase, erektile Dysfunktion und weibliche sexuelle Dysfunktion, daneben aber auch die Prävention und/oder Behandlung von inflammatorischen Erkrankungen, wie z.B. entzündliche Dermatosen (Psoriasis, Akne, Ekzeme, Neurodermitis, Dermatitis, Keratitis, Narbenbildung, Warzenbildung, Frostbeulen), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), von Schmerzzuständen, Krebserkrankungen (Hauthrebs, Liposarcome, Karzinome des Magen-Darm-Traktes, der Leber, Bauchspeicheldrüse, Lunge, Niere, Harnleiter, Prostata und des Genitaltraktes) sowie von Übelkeit und Erbrechen in Verbindung mit Krebstherapien.

Weitere Indikationsgebiete sind beispielsweise die Prävention und/oder Behandlung von Entzündungs- und Immunerkrankungen (Morbus Crohn, Colitis ulcerosa, Lupus erythematodes, rheumatoide Arthritis) und von Erkrankungen der Atemwege, wie beispielsweise chronisch-obstruktive Atemwegserkrankungen (chronische Bronchitis, COPD), Asthma, Lungenemphysem, Bronchiektasien, zystische Fibrose (Mukoviszidose) und pulmonale Hypertonie, insbesondere pulmonale arterielle Hypertonie.

Schließlich kommen die erfindungsgemäßen Verbindungen auch für die Prävention und/ oder Behandlung von Diabetes, insbesondere Diabetes mellitus, Gestationsdiabetes, Insulin-abhängiger Diabetes und Nicht-Insulin-abhängiger Diabetes, von diabetischen Folgeerkrankungen wie z.B. Retinopathie, Nephropathie und Neuropathie, von metabolischen Erkrankungen (Metabolisches Syndrom, Hyperglykämie, Hyperinsulinämie, Insulinresistenz, Glukose-Intoleranz, Fettsucht (Adipositas)) sowie von Arteriosklerose und Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien), insbesondere von Diabetes, Metabolischem Syndrom und Dyslipidämien, in Betracht

Darüber hinaus können die erfindungsgemäßen Verbindungen auch zur Behandlung und/oder Prävention von Schilddrüsenerkrankungen (Hyperthyreose), Erkrankungen der Bauchspeicheldrüse (Pankreatitis), Leberfibrose, viralen Erkrankungen (HPV, HCMV, HIV), Kachexie, Osteoporose, Gicht, Inkontinenz sowie zur Wundheilung und Angiogenese eingesetzt werden.

Weiterer Gegenstand der vorliegenden Erfindung sind die erfindungsgemäßen Verbindungen zur Verwendung in einem Verfahren zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe von koronarer Herzerkrankung, akutem Koronarsyndrom, Angina pectoris, Herzinsuffizienz, Myokardinfarkt und Vorhofflimmern.

Weiterer Gegenstand der vorliegenden Erfindung sind die erfindungsgemäßen Verbindungen zur Verwendung in einem Verfahren zur Behandlung und/oder Prophylaxe von Diabetes, Metabolischem Syndrom und Dyslipidämien.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Verwendung in der Behandlung und/oder Prävention der zuvor genannten Erkrankungen.

Als geeignete Kombinationswirkstoffe seien beispielhaft und vorzugsweise genannt: den Fettstoffwechsel verändernde Wirkstoffe, Antidiabetika, Blutdruck-Senker, durchblutungsfördernd und/ oder antithrombotisch wirkende Mittel, Antioxidantien, Chemokin-Rezeptor-Antagonisten, p38-Kinase-Inhibitoren, NPY-Agonisten, Orexin-Agonisten, Anorektika, PAF-AH-Inhibitoren, Antiphlogistika (COX-Inhibitoren, LTB₄-Rezeptor-Antagonisten), Analgetika wie beispielsweise Aspirin, Anti-Depressiva und andere Pyschopharmaka.

Gegenstand der vorliegenden Erfindung sind insbesondere Kombinationen mindestens einer der erfindungsgemäßen Verbindungen mit mindestens einem den Fettstoffwechsel verändernden Wirkstoff, einem Antidiabetikum, einem blutdrucksenkenden Wirkstoff und/oder einem antithrombotisch wirkenden Mittel.

Die erfindungsgemäßen Verbindungen können vorzugsweise mit einem oder mehreren
- den Fettstoffwechsel verändernden Wirkstoffen, beispielhaft und vorzugsweise aus der Gruppe der HMG-CoA-Reduktase-Inhibitoren, Inhibitoren der HMG-CoA-Reduktase-Expression, Squalensynthese-Inhibitoren, ACAT-Inhibitoren, LDL-Rezeptor-Induktoren, Cholesterin-Absorptionshenuner, polymeren Gallensäureadsorber, Gallensäure-Reabsorptionshemmer, MTP-Inhibitoren, Lipase-Inhibitoren, LpL-Aktivatoren, Fibrate, Niacin, CETP-Inhibitoren, PPAR-α-, PPAR-γ- und/oder PPAR-δ-Agonisten, RXR-Modulatoren, FXR-Modulatoren, LXR-Modulatoren, Thyroidhormone und/oder Thyroidmimetika, ATP-Citrat-Lyase-Inhibitoren, Lp(a)-Antagonisten, Cannabinoid-Rezeptor 1-Antagonisten, Leptin-Rezeptor-Agonisten, Bombesin-Rezeptor-Agonisten, Histamin-Rezeptor-Agonisten sowie der Antioxidantien/Radikalfänger;
- Antidiabetika, die in der Roten Liste 2004/II, Kapitel 12 genannt sind, sowie beispielhaft und vorzugsweise jenen aus der Gruppe der Sulphonylharnstoffe, Biguanide, Meglitinid-Derivate, Glukosidase-Inhibitoren, Inhibitoren der Dipeptidyl-Peptidase IV (DPP-IV-Inhibitoren), Oxadiazolidinone, Thiazolidindione, GLP 1-Rezeptor-Agonisten, Glukagon-Antagonisten, Insulin-Sensitizer, CCK 1-Rezeptor-Agonisten, Leptin-Rezeptor-Agonisten, Inhibitoren von Leberenzymen, die an der Stimulation der Glukoneogenese und/ oder Glykogenolyse beteiligt sind, Modulatoren der Glukoseaufnahme sowie der Kaliumkanalöffner, wie z.B. denjenigen, die in WO 97/26265 und WO 99/03861 offenbart sind;
- den Blutdruck senkenden Wirkstoffen, beispielhaft und vorzugsweise aus der Gruppe der Calcium-Antagonisten, Angiotensin AII-Antagonisten, ACE-Hemmer, Renin-Inhibitoren, beta-Rezeptoren-Blocker, alpha-Rezeptoren-Blocker, Aldosteron-Antagonisten, Mineralocorticoid-Rezeptor-Antagonisten, ECE-Inhibitoren, ACE/NEP Inhibitoren sowie der Vasopeptidase-Inhibitoren; und/oder
- antithrombotisch wirkenden Mitteln, beispielhaft und vorzugsweise aus der Gruppe der Thrombozytenaggregationshemmer oder der Antikoagulantien
- Diuretika;
- Vasopressin-Rezeptor-Antagonisten;
- organischen Nitraten und NO-Donatoren;
- positiv-inotrop wirksamen Verbindungen;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil sowie PDE 3-Inhibitoren wie Milrinone;
- natriuretischen Peptiden, wie z.B. "atrial natriuretic peptide" (ANP, Anaritide), "B-type natriuretic peptide" oder "brain natriuretic peptide" (BNP, Nesiritide), "C-type natriuretic peptide" (CNP) sowie Urodilatin;
- Agonisten des Prostacyclin-Rezeptors (IP-Rezeptors), wie beispielsweise Iloprost, Beraprost, Cicaprost;
- Hemmer des If (funny channel) Kanals, wie beispielsweise Ivabradine;
- Calcium-Sensitizern, wie beispielhaft und vorzugsweise Levosimendan;
- Kalium-Supplements;
- NO-unabhängigen, jedoch Häm-abhängigen Stimulatoren der Guanylatcyclase, wie insbesondere den in WO 00/06568, WO 00/06569, WO 02/42301 und WO 03/095451 beschriebenen Verbindungen;
- NO- und Häm-unabhängigen Aktivatoren der Guanylatcyclase, wie insbesondere den in WO 01/19355, WO 01/19776, WO 01/19778, WO 01/19780, WO 02/070462 und WO 02/070510 beschriebenen Verbindungen;
- Inhibitoren der humanen neutrophilen Elastase (HNE), wie beispielsweise Sivelestat und DX-890 (Reltran);
- die Signaltransduktionskaskade inhibierenden Verbindungen, wie beispielsweise Tyrosinkinase-Inhibitoren, insbesondere Sorafenib, Imatinib, Gefitinib und Erlotinib; und/oder
- den Energiestoffwechsel des Herzens beeinflussenden Verbindungen, wie beispielweise Etomoxir, Dichloracetat, Ranolazine und Trimetazidine
kombiniert werden.

Unter den Fettstoffwechsel verändernden Wirkstoffen werden vorzugsweise Verbindungen aus der Gruppe der HMG-CoA-Reduktase-Inhibitoren, Squalensynthese-Inhibitoren, ACAT-Inhibitoren, Cholesterin-Absorptionshemmer, MTP-Inhibitoren, Lipase-Inhibitoren, Thyroidhormone und/oder Thyroidmimetika, Niacin-Rezeptor-Agonisten, CETP-Inhibitoren, PPAR-α-Agonisten, PPAR-γ-Agonisten, PPAR-δ-Agonisten, polymeren Gallensäureadsorber, Gallensäure-Reabsorptionshemmer, Antioxidantien/Radikalfänger sowie der Cannabinoid-Rezeptor 1-Antagonisten verstanden.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, E-flucimibe oder SMP-797, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem Thyroidhormon und/oder Thyroidmimetikum, wie beispielhaft und vorzugsweise D-Thyroxin oder 3,5,3'-Triiodothyronin (T3), verabreicht.

Bei einer bevorzugten Ausführungsform der Erfindung werden die erfmdungsgemäßen Verbindungen in Kombination mit einem Agonisten des Niacin-Rezeptors, wie beispielhaft und vorzugsweise Niacin, Acipimox, A-cifran oder Radecol, verabreicht.

Bei einer bevorzugten Ausführungsform der Erfindung werden die erfindungsgemäßen Verbindungen in Kombination mit einem CETP-Inhibitor, wie beispielhaft und vorzugsweise Torcetrapib, JTT-705, BAY 60-5521, BAY 78-7499 oder CETP vaccine (Avant), verabreicht.

Bei einer bevorzugten Ausführungsform der Erfindung werden die erfindungsgemäßen Verbindungen in Kombination mit einem PPAR-γ-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-δ-Agonisten, wie beispielhaft und vorzugsweise GW-501516 oder BAY 68-5042, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Antioxidans/Radikalfänger, wie beispielhaft und vorzugsweise Probucol, AGI-1067, BO-653 oder AEOL-10150, verabreicht.

Bei einer bevorzugten Ausführungsform der Erfindung werden die erfindungsgemäßen Verbindungen in Kombination mit einem Cannabinoid-Rezeptor 1-Antagonisten, wie beispielhaft und vorzugsweise Rimonabant oder SR-147778, verabreicht.

Unter Antidiabetika werden vorzugsweise Insulin und Insulinderivate sowie oral wirksame hypoglykämische Wirkstoffe verstanden. Insulin und Insulinderivate umfasst hierbei sowohl Insuline tierischen, menschlichen oder biotechnologischen Ursprungs als auch Gemische hieraus. Die oral wirksamen hypoglykämischen Wirkstoffe umfassen vorzugsweise Sulphonylhamstoffe, Biguanide, Meglitinid-Derivate, Glukosidase-Inhibitoren und PPAR-gamma-Agonisten.

Bei einer bevorzugten Ausführungsform der Erfindung werden die erfindungsgemäßen Verbindungen in Kombination mit Insulin verabreicht.

Bei einer bevorzugten Ausführungsform der Erfindung werden die erfindungsgemäßen Verbindungen in Kombination mit einem Sulphonylharnstoff, wie beispielhaft und vorzugsweise Tolbutamid, Glibenclamid, Glimepirid, Glipizid oder Gliclazid, verabreicht.

Bei einer bevorzugten Ausführungsform der Erfindung werden die erfindungsgemäßen Verbindungen in Kombination mit einem Biguanid, wie beispielhaft und vorzugsweise Metformin, verabreicht.

Bei einer bevorzugten Ausführungsform der Erfindung werden die erfindungsgemäßen Verbindungen in Kombination mit einem Meglitinid-Derivat, wie beispielhaft und vorzugsweise Repaglinid oder Nateglinid, verabreicht.

Bei einer bevorzugten Ausführungsform der Erfindung werden die erfindungsgemäßen Verbindungen in Kombination mit einem Glukosidase-Inhibitor, wie beispielhaft und vorzugsweise Miglitol oder Acarbose, verabreicht.

Bei einer bevorzugten Ausführungsform der Erfindung werden die erfindungsgemäßen Verbindungen in Kombination mit einem DPP-IV-Inhibitor, wie beispielhaft und vorzugsweise Sitagliptin und Vildagliptin, verabreicht.

Bei einer bevorzugten Ausführungsform der Erfindung werden die erfindungsgemäßen Verbindungen in Kombination mit einem PPAR-gamma-Agonisten beispielsweise aus der Klasse der Thiazolidindione, wie beispielhaft und vorzugsweise Pioglitazone oder Rosiglitazone, verabreicht.

Unter den Blutdruck senkenden Mitteln werden vorzugsweise Verbindungen aus der Gruppe der Calcium-Antagonisten, Angiotensin AII-Antagonisten, ACE-Hemmer, beta-Rezeptoren-Blocker, alpha-Rezeptoren-Blocker und Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Valsartan, Candesartan, Embusartan, Olmesartan oder Telmisartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem alpha-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Bei einer bevorzugten Ausführungsform der Erfindung werden die erfindungsgemäßen Verbindungen in Kombination mit einem Aldosteron- oder Mineralokortikoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Vasopressin-Rezeptor-Antagonisten, wie beispielhaft und vorzugsweise Conivaptan, Tolvaptan, Lixivaptan oder SR-121463, verabreicht.

Bei einer bevorzugten Ausführungsform der Erfindung werden die erfindungsgemäßen Verbindungen in Kombination mit einem organischen Nitrat oder NO-Donator, wie beispielhaft und vorzugsweise Natriumnitroprussid, Nitroglycerin, Isosorbidmononitrat, Isosorbiddinitrat, Molsidomin oder SIN-1, oder in Kombination mit inhalativem NO verabreicht.

Bei einer bevorzugten Ausführungsform der Erfindung werden die erfindungsgemäßen Verbindungen in Kombination mit einer positiv-inotrop wirksamen Verbindung, wie beispielhaft und vorzugsweise Herzglycosiden (Digoxin), beta-adrenergen und dopaminergen Agonisten wie Isoproterenol, Adrenalin, Noradrenalin, Dopamin oder Dobutamin, verabreicht.

Bei einer bevorzugten Ausführungsform der Erfindung werden die erfindungsgemäßen Verbindungen in Kombination mit Antisympathotonika wie Reserpin, Clonidin oder alpha-Methyl-Dopa, mit Kaliumkanal-Agonisten wie Minoxidil, Diazoxid, Dihydralazin oder Hydralazin, oder mit Stickoxid freisetzenden Stoffen wie Glycerinnitrat oder Nitroprussidnatrium verabreicht.

Unter antithrombotisch wirkenden Mitteln werden vorzugsweise Verbindungen aus der Gruppe der Thrombozytenaggregationshemmer oder der Antikoagulantien verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Besonders bevorzugt im Rahmen der vorliegenden Erfindung sind Kombinationen enthaltend mindestens eine der erfindungsgemäßen Verbindungen sowie einen oder mehrere weitere Wirkstoffe ausgewählt aus der Gruppe bestehend aus HMG-CoA-Reduktase-Inhibitoren (Statine), Diuretika, beta-Rezeptoren-Blocker, organische Nitrate und NO-Donatoren, ACE-Inhibitoren, Angiotensin AII-Antagonisten, Aldosteron- und Mineralokortikoid-Rezeptor-Antagonisten, Vasopressin-Rezeptor-Antagonisten, Thrombozytenaggregationshemmer und Anti-koagulantien, sowie solche Kombinationen zur Verwendung in der Behandlung und/oder Prävention der zuvor genannten Erkrankungen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie Arzneimittel zur Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- aq.: wässrig
- br s: breites Singulett (bei NMR)
- Bsp.: Beispiel
- c: Konzentration
- d: Dublett (bei NMR)
- dd: Dublett von Dublett (bei NMR)
- DC: Dünnschichtchromatographie
- DCI: direkte chemische Ionisation (bei MS)
- DMF: *N*,*N*-Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ent: Enantiomer / enantiomerenrein
- ESI: Elektrospray-Ionisation (bei MS)
- Et: Ethyl
- Fp.: Schmelzpunkt
- GC-MS: Gaschromatographie-gekoppelte Massenspektrometrie
- h: Stunde(n)
- HATU: *O*-(7-Azabenzotriazol-1-yl)-*N*,*N*,*N'*,*N'*-tetramethyluronium-Hexafluorphosphat
- HPLC: Hochdruck-, Hochleistungsflüssigchromatographie
- kat.: katalytisch
- konz.: konzentriert
- LC-MS: Flüssigchromatographie-gekoppelte Massenspektrometrie
- Lit.: Literatur(stelle)
- MeCN: Acetonitril
- min: Minute(n)
- MS: Massenspektrometrie
- NMP: N-Methylpyrrolidon
- NMR: Kernresonanzspektrometrie
- q: Quartett (bei NMR)
- rac.: racemisch
- RP-HPLC: reverse phase HPLC
- RT: Raumtemperatur
- Rₜ: Retentionszeit (bei HPLC)
- s: Singulett (bei NMR)
- t: Triplett (bei NMR)
- t-Bu: tert.-Butyl
- TFA: Trifluoressigsäure
- THF: Tetrahydrofuran
- verd.: verdünnt

### HPLC-, LC-MS- und GC-MS-Methoden:

Methode 1 (HPLC): Instrument: Hewlett Packard Series 1050; Säule: Symmetry TM C18 3.9 x 150 mm; Fluss: 1.5 ml/min; Eluent A: Wasser, Eluent B: Acetonitril; Gradient: → 0.6 min 10% B → 3.8 min 100% B → 5.0 min 100% B → 5.5 min 10% B; Stopzeit: 6.0 min; Injektionsvolumen: 10 µl; Diodenarraydetektor-Signal: 214 und 254 nm.

Methode 2 (LC-MS): Gerätetyp MS: Micromass ZQ; Gerätetyp HPLC: Waters Alliance 2795; Säule: Merck Chromolith SpeedROD RP-18e 100 mm x 4.6 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 10% B → 7.0 min 95% B → 9.0 min 95% B; Ofen: 35°C; Fluss: 0.0 min 1.0 ml/min → 7.0 min 2.0 ml/min → 9.0 min 2.0 ml/min; UV-Detektion: 210 nm.

Methode 3 (LC-MS): Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min. 2 ml/min; Ofen: 50°C; UV-Detektion: 210 nm.

Methode 4 (LC-MS): Instrument: Micromass Quattro LCZ mit HPLC Agilent Serie 1100; Säule: Phenomenex Onyx Monolithic C18, 100 mm x 3 mm. Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2 min 65% A → 4.5 min 5% A → 6 min 5% A; Fluss: 2 ml/min; Ofen: 40°C; UV-Detektion: 208- 400 nm.

Methode 5 (LC-MS): Gerätetyp MS: Micromass ZQ; Gerätetyp HPLC: Waters Alliance 2795; Säule: Phenomenex Synergi 2.5 µ MAX-RP 100A Mercury 20 mm x 4mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

Methode 6 (LC-MS): Instrument: Micromass QuattroPremier mit Waters UPLC Acquity ; Säule: Thermo Hypersil GOLD 1,9µ 50x 1mm; Eluent A: 1 1 Wasser + 0.5ml 50%ige Ameisensäure, Eluent B: 1 1 Acetonitril + 0.5ml 50%ige Ameisensäure; Gradient: 0.0 min 90% A → 0.1 min 90% A → 1.5 min 10% A → 2.2 min 10% A; Ofen:50°C; Fluss: 0.33 ml/min; UV-Detektion: 210 nm.

Methode 7 (LC-MS): Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

Methode 8 (LC-MS): Instrument: Micromass Quattro LCZ mit HPLC Agilent Serie 1100; Säule: Phenomenex Synergi 2.5 µ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 0.1 min 90% A → 3.0 min 5% A → 4.0 min 5% A → 4.1 min 90% A; Fluss: 2 ml/min; Ofen: 50°C; UV-Detektion: 208- 400 nm.

Methode 9 (LC-MS): Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90% A → 2.5 min 30% A → 3.0 min 5% A → 4.5 min 5% A; Fluss: 0.0 min 1 ml/min, 2.5 min/3.0 min/4.5 min 2 ml/min; Ofen: 50°C; UV-Detektion: 208-400 nm.

Methode 10 (LC-MS): Gerätetyp MS: Micromass ZQ; Gerätetyp HPLC: Waters Alliance 2795; Säule: Merck Chromolith SpeedROD RP-18e 100 x 4.6mm; Eluent A: 1 1 Wasser + 0.5 ml 50%ige Ameisensäure; Eluent B: 1 1 Acetonitril + 0.5 ml 50%ige Ameisensäure; Gradient: 0.0 min 10% B→ 7.0 min 95% B→ 9.0 min 95% B; Ofen: 35°C; Fluss: 0.0 min 1.0 ml/min→ 7.0 min 2.0 ml/min→ 9.0 min 2.0 ml/min; UV-Detektion: 210 nm.

Methode 11 (LC-MS): Instrument: Micromass Platform LCZ mit HPLC Agilent Serie 1100; Säule: Thermo Hypersil GOLD 3µ 20x 4mm; Eluent A: 1 1 Wasser + 0.5ml 50%ige Ameisensäure , Eluent B: 1 1 Acetonitril + 0.5ml 50%ige Ameisensäure; Gradient: 0.0 min 100% A → 0.2 min 100% A → 2.9 min 30% A → 3.1 min 10% A →5.5 min 10% A; Ofen:50°C; Fluss: 0.8ml/min; UV-Detektion: 210 nm.

Methode 12 (LC-MS): Gerätetyp MS: M-40 DCI (NH₃); Gerätetyp HPLC: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

Methode 13 (LC-MS): Instrument: Micromass Quattro Micro MS mit HPLC Agilent Serie 1100; Säule: Thermo Hypersil GOLD 3µ 20 x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 100% A→ 3.0 min 10% A → 4.0 min 10% A → 4.01 min 100% A (Fluss 2.5ml) →5.00 min 100% A; Ofen: 50°C; Fluss: 2 ml/min; UV-Detektion: 210nm

### Ausgangsverbindungen und Intermediate:

### Beispiel 1A

### 2-Amino-4-[4-(2-hydroxyethoxy)phenyl]-6-sulfanylpyridin-3,5-dicarbonitril

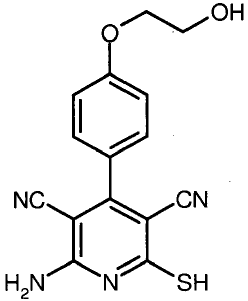

Die Darstellung wurde, wie in WO 03/053441 für Beispiel 6 (1. Stufe) beschrieben, durchgeführt.
LC-MS (Methode 4): Rₜ = 1.73 min; MS (ESIpos): m/z = 313 [M+H]⁺.

### Beispiel 2A

### 2-Amino-6-({[2-(4-chlorphenyl)-1,3-thiazol-4-yl]methyl}sulfanyl)-4-[4-(2-hydroxyethoxy)phenyl]pyridin-3,5-dicarbonitril

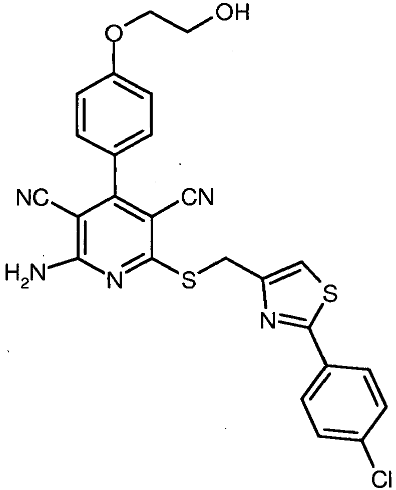

Die Darstellung wurde, wie in WO 03/053441 für Beispiel 6 (2. Stufe) beschrieben, durchgeführt.
LC-MS (Methode 10): Rₜ = 5.69 min; MS (ESIpos): m/z = 520 [M+H]⁺.

### Beispiel 3A

### 2-Amino-4-phenyl-6-sulfanylpyridin-3,5-dicarbonitril

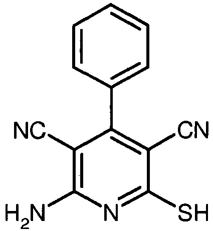

Die Darstellung wurde, wie in WO 03/053441 für Beispiel 6 (1. Stufe) beschrieben, durchgeführt
MS (ESIpos): m/z = 253 (M+H)⁺

### Beispiel 4A

### 2-Amino-6-({[2-(4-chlorphenyl)-1,3-thiazol-4-yl]methyl}sulfanyl)-4-phenylpyridin-3,5-dicarbonitril

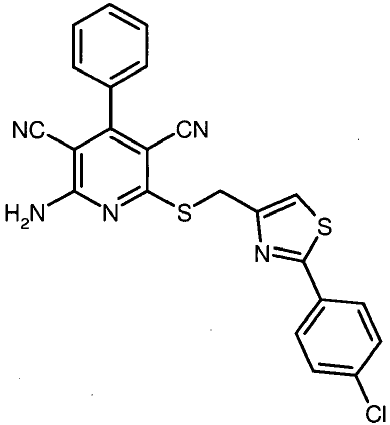

5.0 g (19.82 mmol) der Verbindung aus Beispiel 3A, 5.0 g (59.45 mmol) Natriumhydrogencarbonat und 5.32 g (21.80 mmol) 4-(Chlormethyl)-2-(4-chlorphenyl)-1,3-thiazol wurden in 100 ml absolutem DMF zusammengegeben und über Nacht bei Raumtemperatur gerührt. Das Reaktionsgemisch wurde auf 700 ml Wasser gegossen. Der entstandene Niederschlag wurde über eine Glasfritte abfiltriert und mit Wasser gewaschen. Der Rückstand wurde im Vakuum getrocknet.
Ausbeute: 9.25 g (93% d. Th., 92% Reinheit)
LC-MS (Methode 4): Rₜ = 4.26 min; MS (ESIpos): m/z = 460 [M+H]⁺.

### Beispiel 7A

### 2-{4-[2-Amino-6-({[2-(4-chlorphenyl)-1,3-thiazol-4-yl]methyl}sulfanyl)-3,5-dicyanopyridin-4-yl]phenoxy}ethylacetat

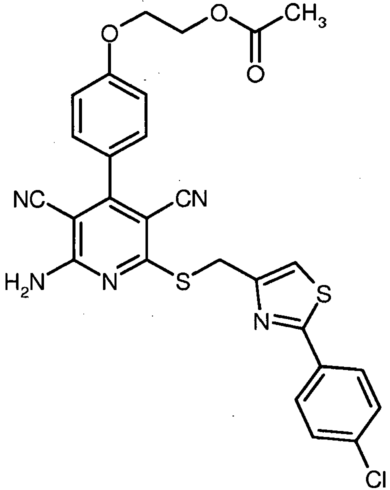

500 mg (0.96 mmol) der Verbindung aus Beispiel 2A wurden in 1.9 ml Essigsäureanhydrid gelöst und 1 h zum Rückfluss erhitzt. Der Ansatz wurde mit ca. 1 ml 1N Salzsäure versetzt und zehn Minuten nachgerührt. Der entstehende Niederschlag wurde abfiltriert und in 8 ml Ethanol aufgenommen, kurz gerührt und nochmals abfiltriert. Der Niederschlag wurde im Vakuum getrocknet.
Ausbeute: 422 mg (77% d. Th.)
¹H-NMR (400 MHz, DMSO-d₆): δ = 7.96 (s, 1H), 7.93 (d, 2H), 7.57 (d, 2H), 7.49 (d, 2H), 7.11 (d, 2H), 4.63 (s, 2H), 4.38-4.35 (m, 2H), 4.29-4.25 (m, 2H), 2.05 (s, 3H).
LC-MS (Methode 7): Rₜ = 4.08 min; MS (ESIpos): m/z = 562 [M+H]⁺.

### Beispiel 9A

### 7-Chlor-2,4-dioxo-5-phenyl-1,2,3,4-tetrahydropyrido[2,3-d]pyrimidin-6-carbonitril

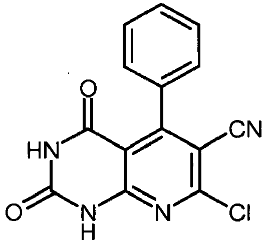

1.0 g (3.04 mmol) 7-Amino-2,4-dioxo-5-phenyl-1,2,3,4-tetrahydropyrido[2,3-d]pyrimidin-6-carbonitril [Assy, M. G. et al., J. Indian Chem. Soc. 1996, 73 (11), 623-624] wurden in 8 ml DMSO gelöst und mit 0.82 ml (713 mg, 6.09 mmol) Isopentylnitrit und 818 mg (6.09 mmol) Kupfer(II)-chlorid versetzt und über Nacht bei 80°C gerührt. Die Reaktionsmischung wurde abgekühlt und anschließend mit 6 ml 1 N Salzsäure-Lsg. und dann mit 100 ml Wasser versetzt. Der entstandene Niederschlag wurde abfiltriert und im Hochvakuum getrocknet. Es erfolgte keine weitere Reinigung (LC-MS-Reinheit: ca. 82%).
Ausbeute: 729 mg (64% d. Th.)
¹H-NMR (400 MHz, DMSO-d₆): δ = 12.45 (br s, 1H), 11.59 (s, 1H), 7.50-7.43 (m, 3H), 7.36-7.29 (m, 2H). LC-MS (Methode 3): Rₜ = 1.94 min; MS (ESIpos): m/z = 299 [M+H]⁺.

### Beispiel 10A

### 7-Amino-5-[4-(2-hydroxyethoxy)phenyl]-2,4-dioxo-1,2,3,4-tetrahydropyrido[2,3-d]pyrimidin-6-carbonitril

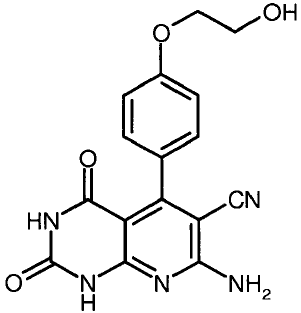

100 mg (0.602 mmol) 4-(2-Hydroxyethoxy)benzaldehyd, 76 mg (0.602 mmol) 6-Amino-1,2,3,4-tetrahydropyrimidin und 40 mg (0.602 mmol) Malonsäuredinitril wurden in 2 ml Ethanol vorgelegt, mit 0.13 ml (122 mg, 1.204 mmol) 4-Methylmorpholin versetzt und über Nacht bei Rückfluss gerührt. Die Reaktionsmischung wurde abgekühlt und anschließend mit ca. 20 ml Wasser versetzt. Der entstandene Niederschlag wurde abfiltriert und getrocknet. Das Rohprodukt ohne weitere Reinigung in der Folgereaktion eingesetzt.
Ausbeute: 128 mg (Reinheit ca. 50%)
LC-MS (Methode 5): Rₜ = 0.74 min; MS (ESIpos): m/z = 340 [M+H]⁺.

### Beispiel 11A

### 7-Chlor-5-[4-(2-hydroxyethoxy)phenyl]-2,4-dioxo-1,2,3,4-tetrahydropyrido[2,3-d]pyrimidin-6-carbonitril

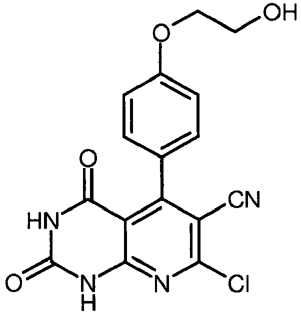

863 mg (ca. 1.272 mmol) Beispiel 10A wurden in 12 ml DMSO/Acetonitril (1:1) vorgelegt und mit 0.34 ml (298 mg, 2.54 mmol) Isopentylnitrit und 342 mg (2.54 mmol) Kupfer(II)-chlorid versetzt und 4 h bei 80°C gerührt. Die Reaktionsmischung wurde abgekühlt und anschließend mit 2.5 ml 1 N Salzsäure-Lsg. und dann mit ca. 50 ml Wasser versetzt. Der entstandene Niederschlag wurde abfiltriert und das Filtrat wurde eingedampft. Die Rohprodukt wurde über eine präparative HPLC (Chromasil, Wasser/Acetonitril +0.1% TFA) gereinigt.
Ausbeute: 143 mg (31 % d. Th.)
LC-MS (Methode 3): Rₜ = 1.54 min; MS (ESIpos): m/z = 359 [M+H]⁺.

### Beispiel 12A

### 4-(Chlormethyl)-2-(4-chlorphenyl)-1,3-oxazol

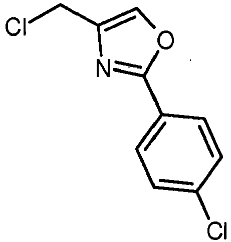

408 mg (3.21 mmol) 1,3-Dichloraceton und 500 mg (3.21 mmol) 4-Chlorbenzamid wurden vereint und 1 h bei 135 °C gerührt. Anschließend wurde auf RT abgekühlt und vorsichtig mit 1.1 ml konz. Schwefelsäure versetzt und 5 min nachgerührt. Das Gemisch wurde vorsichtig auf Eis gegossen. Der Niederschlag wurde abfiltriert und mit Wasser nachgewaschen. Nach Trocknen wurde das Rohprodukt ohne weitere Reinigung in der Folgereaktion eingesetzt.
Ausbeute: 426 mg (49% d. Th., 85% Reinheit)
LC-MS (Methode 11): Rₜ = 3.78 min; MS (ESIpos): m/z = 228 [M].

### Beispiel 13A

### 2-(4-Chlorphenyl)-4,5-dimethyl-1,3-oxazol-3-oxid

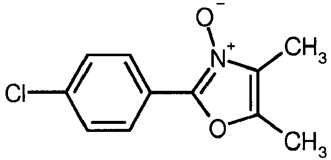

1.00 g (9.89 mmol) Diacetylmonoxim und 1.53 g (10.88 mmol) 4-Chlorbenzaldehyd wurden in 2 ml (34.94 mmol) Eisessig vorgelegt. Dann wurde 30 min lang Chlorwasserstoff-Gas unter Eiskühlung des Reaktionsgemisches eingeleitet. Anschließend wurde das Reaktionsgemisch mit 10 ml Diethylether versetzt. Es fällt ein Niederschlag aus, der abfiltriert und zweimal mit je 2 ml Diethylether gewaschen wurde. Der Niederschlag wurde in ca. 5 ml Wasser re-suspendiert und die Suspension mit 25%iger wässriger Ammoniaklösung basisch gestellt. Es wurde dann viermal mit je 10 ml Dichlormethan extrahiert. Die vereinigten organischen Phasen wurden über Magnesiumsulfat getrocknet und das Lösungsmittel am Rotationsverdampfer entfernt. Der Rückstand wurde ohne weitere Reinigung in der Folgereaktion eingesetzt.
Ausbeute: 1.85 g (84% d. Th.)
LC-MS (Methode 5): Rₜ = 2.29 min; MS (ESIpos): m/z = 224 [M+H]⁺.

### Beispiel 14A

### 4-(Chlormethyl)-2-(4-chlorphenyl)-5-methyl-1,3-oxazol

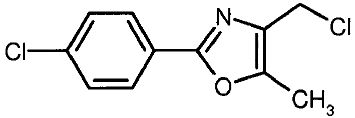

1.00 g (4.47 mmol) der Verbindung aus Beispiel 13A wurden in 15 ml Chloroform vorgelegt und vorsichtig mit 1.5 ml (16.10 mmol) Phosphoroxychlorid versetzt. Das Reaktionsgemisch wurde 30 min unter Rühren zum Rückfluss erhitzt. Der Ansatz wurde anschließend auf 0°C abgekühlt und durch Zugabe von 25%iger wässriger Ammoniaklösung schwach basisch gestellt. Das Gemisch wurde dreimal mit je 20 ml Ethylacetat extrahiert. Die vereinigten organischen Phasen wurden zweimal mit je 5 ml Wasser gewaschen und anschließend über Magnesiumsulfat getrocknet. Das Lösungsmittel wurde am Rotationsverdampfer entfernt. Der Rückstand wurde ohne weitere Reinigung in den Folgestufen eingesetzt.
Ausbeute: 1.33 g (96% d. Th., 78% Reinheit)
¹H-NMR (400 MHz, DMSO-d₆): δ = 7.95 (d, 2H), 7.60 (d, 2H), 4.77 (s, 2H), 2.44 (s, 3H).
LC-MS (Methode 3): Rₜ = 2.80 min; MS (ESIpos): m/z = 242 [M+H]⁺.

### Beispiel 15A

### 2-Amino-6-({[2-(4-chlorphenyl)-1,3-oxazol-4-yl]methyl}sulfanyl)-4-[4-(2-hydroxyethoxy)phenyl]pyridin-3,5-dicarbonitril

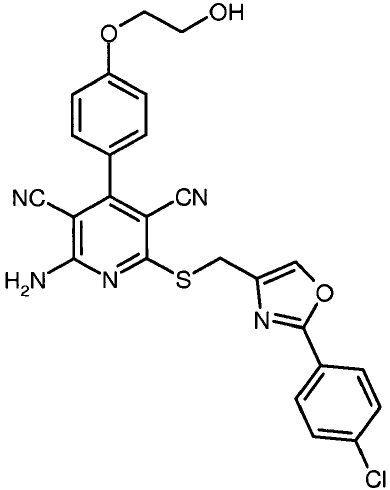

500 mg (1.60 mmol) Beispiel 1A, 365 mg (1.60 mmol) Beispiel 12A und 403 mg (4.80 mmol) Natriumhydrogencarbonat wurden in 11 ml trockenem DMF gelöst. Das Reaktionsgemisch wurde 2 h bei RT gerührt. Der Ansatz wurde mit ca. 5 ml Wasser verdünnt und 1 h nachgerührt. Der entstandene Niederschlag wurde abfiltriert und bei 40°C im Trockenschrank getrocknet. Eine weitere Reinigung ist mittels präparativer HPLC (Säule: YMC GEL ODS-AQ S-5 / 15 µm; Laufmittelgradient: Acetonitril/Wasser 10:90 → 95:5) möglich.
Ausbeute: 665 mg (77% d. Th.)
¹H-NMR (400 MHz, DMSO-d₆): δ = 8.37 (s, 1H), 8.31-7.89 (br. s, 2H), 7.97 (d, 2H), 7.60 (d, 2H), 7.46 (d, 2H), 7.10 (d, 2H), 4.91 (t, 1H), 4.41 (s, 2H), 4.08 (t, 2H), 3.74 (q, 2H).
LC-MS (Methode 3): Rₜ = 2.53 min; MS (ESIpos): m/z = 504 [M+H]⁺.

### Ausführungsbeispiele:

### Beispiel 11

### 7-({[2-(4-Chlorphenyl)-1,3-thiazol-4-yl]methyl}thio)-2,4-dioxo-5-phenyl-1,2,3,4-tetrahydropyrido[2,3-d]pyrimidin-6-carbonitril

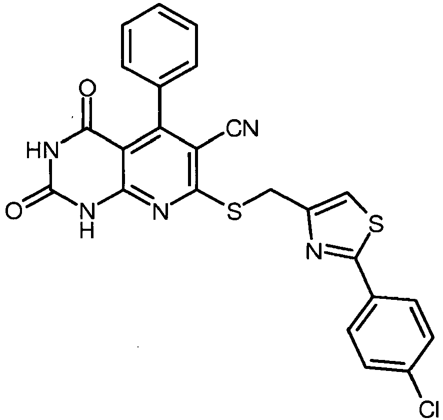

100 mg (0.275 mmol) der Verbindung aus Beispiel 9A wurden in 0.55 ml DMF gelöst und mit 25 mg (0.329 mmol) Natriumsulfid versetzt und über Nacht bei RT gerührt. Die Reaktionslösung wurde anschließend mit 1 ml DMF verdünnt mit 74 mg (0.302 mmol) 4-(Chlormethyl)-2-(4-chlorphenyl)-1,3-thiazol und 69 mg (0.825 mmol) Natriumhydrogencarbonat versetzt und 45 min bei RT gerührt. Die Reaktionsmischung wurde mit Wasser versetzt. Der entstandene Niederschlag wurde mit THF/Methanol (5 ml/2 ml) ausgerührt und anschließend wurde abfiltriert. Es wurden 21 mg (15% d. Th.) des Produktes als Feststoff erhalten. Das Filtrat wurde zur weiteren Reinigung über eine präparative HPLC (Chromasil, Wasser/Acetonitril + 0.1% TFA) gereinigt. Es wurden nochmals 70 mg (50% d. Th.) des Produktes erhalten.
Gesamtausbeute: 91 mg (65% d. Th.)
¹H-NMR (400 MHz, DMSO-d₆): δ = 12.23 (s, 1H), 11.39 (s, 1H), 8.08 (s, 1H), 7.93 (d, 2H), 7.58 (d, 2H), 7.46-7.38 (m, 3H), 7.30-7.27 (m, 2H), 4.72 (s, 2H).
LC-MS (Methode 3): Rₜ = 2.80 min; MS (ESIpos): m/z = 504 [M+H]⁺.

### Beispiel 12

### 7-({[2-(4-Chlorphenyl)-1,3-thiazol-4-yl]methyl}sulfanyl)-5-[4-(2-hydroxyethoxy)phenyl]-2,4-dioxo-1,2,3,4-tetrahydropyrido[2,3-d]pyrimidin-6-carbonitril

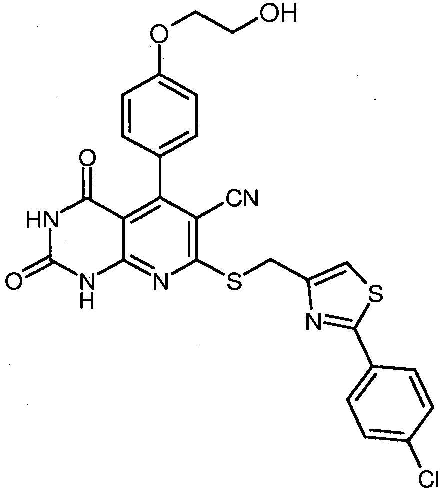

100 mg (0.229 mmol) der Verbindung aus Beispiel 11A wurden in 0.46 ml DMF gelöst und mit 21 mg (0.274 mmol) Natriumsulfid versetzt und über Nacht bei RT gerührt. Die Reaktionslösung wurde anschließend mit 33 mg (0.137 mmol) 4-(Chlormethyl)-2-(4-chlorphenyl)-1,3-thiazol und 23 mg (0.275 mmol) Natriumhydrogencarbonat versetzt und 2 h bei RT gerührt. Die Reaktionsmischung wurde mit Wasser versetzt zur weiteren Reinigung über eine präparative HPLC (Chromasil, Wasser/Acetonitril + 0.1% TFA) gereinigt.
Ausbeute: 39 mg (75% d. Th.)
¹H-NMR (400 MHz, DMSO-d₆): δ = 12.20 (s, 1H), 11.36 (s, 1H), 8.06 (s, 1H), 7.94 (d, 2H), 7.58 (d, 2H), 7.21 (d, 2H), 6.98 (d, 2H), 4.89 (t, 1H), 4.72 (s, 2H), 4.05 (t, 2H), 3.73 (q, 2H).
LC-MS (Methode 6): Rₜ = 1.21 min; MS (ESIpos): m/z = 564 [M+H]⁺.

### B. Bewertung der pharmakologischen und Physiologischen Wirksamkeit

Die pharmakologische und physiologische Wirkung der erfindungsgemäßen Verbindungen kann in folgenden Assays gezeigt werden:

### B-1. Indirekte Bestimmung des Adenosin-Agonismus über Genexpression

Zellen der permanenten Linie CHO (Chinese Hamster Ovary) werden stabil mit der cDNA für die Adenosin-Rezeptor-Subtypen A1, A2a und A2b transfiziert. Die Adenosin-A1-Rezeptoren sind über Gᵢ-Proteine und die Adenosin-A2a- und A2b-Rezeptoren über Gₛ-Proteine an die Adenylatcyclase gekoppelt. Entsprechend wird die cAMP-Bildung in der Zelle inhibiert bzw. stimuliert. Über einen cAMP-abhängigen Promoter wird danach die Expression der Luziferase moduliert. Der Luziferase-Test wird mit dem Ziel hoher Sensitivität und Reproduzierbarkeit, geringer Varianz und guter Eignung für die Durchführung auf einem Robotersystem optimiert durch Variation mehrerer Testparameter, wie z.B. Zelldichte, Dauer der Anzuchtphase und der Testinkubation, Forskolin-Konzentration und Medium-Zusammensetzung. Zur pharmakologischen Charakterisierung der Zellen und zum Roboter-gestützten Substanz-Screening wird das folgende Testprotokoll verwendet:
Die Stammkulturen werden in DMEM/F12-Medium mit 10% FCS (fötales Kälberserum) bei 37°C unter 5% CO₂ gezüchtet und jeweils nach 2-3 Tagen 1:10 gesplittet. Testkulturen werden mit 2000 Zellen pro Napf in 384-well-Platten ausgesät und ca. 48 Stunden bei 37°C angezogen. Dann wird das Medium durch eine physiologische Kochsalzlösung (130 mM Natriumchlorid, 5 mM Kaliumchlorid, 2 mM Calciumchlorid, 20 mM HEPES, 1 mM Magnesiumchlorid-Hexahydrat, 5 mM Natriumhydrogencarbonat, pH 7.4) ersetzt. Die in DMSO gelösten zu testenden Substanzen werden in einer Verdünnungsreihe von 5 x 10⁻¹¹ M bis 3 x 10⁻⁶ M (Endkonzentration) zu den Testkulturen pipettiert (maximale DMSO-Endkonzentration im Testansatz: 0.5%). 10 Minuten später wird Forskolin zu den A1-Zellen zugegeben und anschließend werden alle Kulturen für vier Stunden bei 37°C inkubiert. Danach wird zu den Testkulturen 35 µl einer Lösung, bestehend zu 50% aus Lyse-Reagenz (30 mM Dinatriumhydrogenphosphat, 10% Glycerin, 3% TritonX100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) zugegeben, ca. 1 Minute geschüttelt und die Luciferase-Aktivität mit einem Kamerasystem gemessen. Bestimmt werden die EC₅₀-Werte, d.h. die Konzentrationen, bei denen bei der A1-Zelle 50% der Luciferase-Antwort inhibiert bzw. bei den A2bund A2a-Zellen 50% der maximalen Stimulierbarkeit mit der entsprechenden Substanz erreicht sind. Als Referenzverbindung dient in diesen Experimenten die Adenosin-analoge Verbindung NECA (5-N-Ethylcarboxamido-adenosin), die mit hoher Affinität an alle Adenosin-Rezeptor-Subtypen bindet und eine agonistische Wirkung besitzt [Klotz, K.N., Hessling, J., Hegler, J., Owman, C., Kull, B., Fredholm, B.B., Lohse, M.J., "Comparative pharmacology of human adenosine receptor subtypes - characterization of stably transfected receptors in CHO cells", Naunyn Schmiedebergs Arch. Pharmacol. 357, 1-9 (1998)].

In der folgenden Tabelle 1 sind die EC₅₀-Werte repräsentativer Ausführungsbeispiele für die Rezeptorstimulation an Adenosin A1-, A2a- und A2b-Rezeptor-Subtypen aufgeführt:

**Tabelle 1**

| **Beispiel Nr.** | **EC50 A1 [nM] (1 µM Forskolin)** | **EC50 A2a [nM]** | **EC50 A2b [nM]** |
|---|---|---|---|
| 12 | 2 | 2000 | 880 |

### B-2. Untersuchung an isolierten Gefäßen

Aus narkotisierten Ratten wird die Arteria caudalis präpariert und in eine konventionelle Apparatur zur Messung isolierter Gefäße eingespannt. Die Gefäße werden in einem Wärmebad perfundiert und mit Phenylephrin kontrahiert. Das Maß der Kontraktion wird über einen Kontraktionsmesser ermittelt. Zu den vorkontrahierten Gefäßen werden Testsubstanzen gegeben und die Abnahme der Kontraktion der Gefäße gemessen. Eine Abnahme der Kontraktion entspricht einer Dilatation der Gefäße. Als EC₅₀-Wert einer Testsubstanz bzgl. ihrer relaxierenden Eigenschaften wird die Konzentration angegeben, bei der die Kontraktion der Gefäße um 50% verringert ist.

### B-3. Blutdruck- und Herzfrequenz-Messungen an wachen Ratten

Wachen SHR (spontaneously hypertensive rats)-Ratten, die einen internen Sender tragen, der dauerhaft sowohl Blutdruck als auch Herzfrequenz messen kann (telemetrische Erfassung von hämodynamischen Parametern), werden Testsubstanzen in verschiedenen Dosierungen oral verabreicht. Anschließend werden über 24 Stunden Blutdruck und Herzfrequenz und deren Veränderungen aufgezeichnet.

### B-4. Blutdruck- und Herzfrequenz-Messungen an wachen Krallenaffen

Wachen Krallenaffen, die einen internen Sender tragen, der dauerhaft sowohl Blutdruck als auch Herzfrequenz messen kann (telemetrische Erfassung von hämodynamischen Parametern), werden Testsubstanzen in verschiedenen Konzentrationen oral verabreicht. Anschließend werden über 6-24 Stunden Blutdruck und Herzfrequenz und deren Veränderungen aufgezeichnet.

### B-5. Indirekte Bestimmung des Adenosin-Antagonismus über Genexpression

Zellen der permanenten Linie CHO K1 (Chinese Hamster Ovary) werden stabil mit einem Reporterkonstrukt (CRE-Luciferase) und der cDNA für die Adenosin-Rezeptorsubtypen A2a oder A2b transfiziert. A2a- bzw. A2b-Rezeptoren sind über Gas-Proteine an die Adenylatcyclase gekoppelt. Durch Rezeptoraktivierung wird die Adenylatcyclase aktiviert und damit das cAMP-Level in der Zelle erhöht. Über das Reporterkonstrukt, einem cAMP-abhängigen Promotor, ist die Änderung des cAMP-Levels an die Luciferase-Expression gekoppelt.

Für die Bestimmung von Adenosin-Antagonismus am Adenosin-Rezeptorsubtyp A1 werden ebenfalls CHO K1-Zellen stabil transfiziert, diesmal allerdings mit einem Ca²⁺-sensitiven Reporterkonstrukt (NFAT-TA-Luc; Clontech) und einem A1-Gα16 Fusionskonstrukt. Diese Rezeptorchimäre ist im Gegensatz zum nativen A1-Rezeptor (Gαi-Kopplung) an die Phospholipase C gekoppelt. Die Luciferase wird hier in Abhängigkeit von der cytosolischen Ca²⁺-Konzentration exprimiert.

Die permanenten Zelllinien werden in DMEM/F12 (Cat.-No BE04-687Q; BioWhittaker) mit 10% FCS (Fetales Kälberserum) und diversen Zusätzen (20 ml/Liter 1M HEPES (Cat.-No 15630; Gibco), 20 ml/Liter GlutaMAX (Cat.-No 35050-038, Gibco), 14 ml/Liter MEM Natriumpyruvat (Cat.-No 11360-039; Gibco), 10 ml/Liter PenStrep (Cat.-No 15070-063; Gibco)) bei 37°C unter 5% Kohlendioxid kultiviert und zweimal pro Woche gesplittet.

Für die Testung im 384-well Plattenformat werden die Zellen mit 2000 Zellen/Well in 25µl/Well Aussaatmedium ausgesät und bis zur Substanztestung bei 37°C unter 5% Kohlendioxid kultiviert. Die A2a- und A2b-Zellen werden 24 h vor der Substanztestung in Medium mit Zusätzen und 5% FCS ausgesät, wobei für die A2a-Zellen als Basismedium DMEM/F12 und für die A2b-Zellen OptiMEM (Cat.-No 31985-047; Gibco) verwendet wird. Die A1-Gα16-Zellen werden 48 h vor Substanztestung in OptiMEM mit 2.5% dialysiertem FCS und Zusätzen ausgesät. Am Testtag wird vor der Substanzzugabe das Medium durch 25 µl Cafty-Puffer (Cat.-No T21-154; PAA) mit 2 mM Calciumchlorid und 0.1% BSA (Rinderserumalbumin) ersetzt. Von den zu testenden, in DMSO gelösten Substanzen werden Verdünnungsreihen in Cafty-Puffer mit 2 mM Calciumchlorid und 0.1% BSA (Rinderserumalbumin) und einer geeigneten Konzentration des Agonisten angesetzt. Die Substanzen werden in einer Endkonzentration von 5 x 10⁻⁵ M bis 2.56 x 10⁻¹¹ M zu den Testkulturen pipettiert, wobei der DMSO-Gehalt auf den Zellen 0.5% nicht überschreiten darf. Als Agonist wird für die A2a- und A2b-Zellen NECA (5-N-Ethylcarboxamido-adenosin) in einer Endkonzentration von 30 nM, was etwa der EC₅₀-Konzentration entspricht, eingesetzt. Für die A1-Gα16-Zellen wird 25 nM CPA (N6-cyclopentyl-adenosine) als Agonist eingesetzt, was etwa der EC₇₅-Konzentration entspricht. Nach der Substanzzugabe werden die Zellplatten 3-4 h bei 37°C unter 5% Kohlendioxid inkubiert. Anschließend werden die Zellen direkt vor der Messung mit 25 µl einer Lösung, bestehend zu 50% aus Lyse-Reagenz (30 mM Dinatriumhydrogenphosphat, 10% Glycerin, 3% Triton X-100, 25 mM TrisHCl, 2 mM Dithiotreitol (DTT), pH 7.8) und zu 50% aus Luciferase-Substrat-Lösung (2.5 mM ATP, 0.5 mM Luciferin, 0.1 mM Coenzym A, 10 mM Tricin, 1.35 mM Magnesiumsulfat, 15 mM DTT, pH 7.8) versetzt. Die Luciferase-Aktivität wird mit einem Lumineszenz-Reader detektiert. Bestimmt werden die IC₅₀-Werte, d.h. die Konzentration, bei denen die Luciferase-Antwort, hervorgerufen durch den jeweiligen Agonisten zu 50% inhibiert wird. Als Referenz-Antagonist wird für die A2a- und A2b-Zellen ZM241385 und für die A1-Gα16-Zellen DPCPX (1,3-dipropyl-8-cyclopentylxanthine) verwendet.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 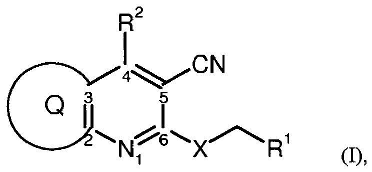 in welcher
der Ring Q für eine Gruppe der Formel 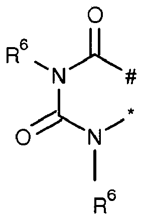 steht, wobei
* die Anknüpfstelle an das C2-Atom bedeutet,
# die Anknüpfstelle an das C3-Atom bedeutet,
R⁶ für jeweils Wasserstoff, (C₁-C₄)-Alkyl oder Allyl steht,
worin (C₁-C₄)-Alkyl mit einem Substituenten ausgewählt aus der Gruppe Hydroxycarbonyl, (C₁-C₄)-Alkoxycarbonyl und Amino substituiert sein kann,
X für S oder O steht,
R¹ für (C₆-C₁₀)-Aryl oder 5- bis 10-gliedriges Heteroaryl steht,
wobei (C₆-C₁₀)-Aryl und 5- bis 10-gliedriges Heteroaryl mit 1 oder 2 Substituenten unabhängig voneinander ausgewählt aus der Gruppe Halogen, Nitro, Cyano, (C₁-C₆)-Alkyl, Trifluormethyl, Hydroxy, (C₁-C₆)-Alkoxy, Amino, Mono-(C₁-C₆)-alkylamino, Di-(C₁-C₆)-alkylamino, Hydroxycarbonyl, (C₁-C₆)-Alkoxycarbonyl, Aminocarbonyl, Mono-(C₁-C₆)-alkylaminocarbonyl, Di-(C₁-C₆)-alkylaminocarbonyl, Pyrrolidino, Piperidino, Morpholino, Piperazino und *N'*-(C₁-C₄)-Alkylpiperazino, Phenyl und 5- oder 6-gliedriges Heteroaryl, substituiert sein können,
worin Phenyl und 5- oder 6-gliedriges Heteroaryl mit 1 bis 3 Substituenten unabhängig voneinander ausgewählt aus der Gruppe Halogen, Nitro, Cyano, (C₁-C₆)-Alkyl, Difluormethyl, Trifluormethyl, Hydroxy, (C₁-C₆)-Alkoxy, Difluormethoxy, Trifluormethoxy, Amino, Mono-(C₁-C₆)-alkylamino, Di-(C₁-C₆)-alkylamino, Hydroxycarbonyl und (C₁-C₆)-Alkoxycarbonyl substituiert sein können,
R² für (C₅-C₆)-Cycloalkyl, 5- oder 6-gliedriges Heterocyclyl, Phenyl oder 5- oder 6-gliedriges Heteroaryl steht,
wobei (C₅-C₆)-Cycloalkyl mit 1 oder 2 Substituenten unabhängig voneinander ausgewählt aus der Gruppe (C₁-C₆)-Alkyl, Hydroxy, Oxo, (C₁-C₆)-Alkoxy, Amino, Mono-(C₁-C₆)-alkylamino und Di-(C₁-C₆)-alkylamino substituiert sein kann,
worin (C₁-C₆)-Alkyl und (C₁-C₆)-Alkoxy mit 1 oder 2 Substituenten unabhängig voneinander ausgewählt aus der Gruppe Hydroxy, (C1-C4)-Alkoxy und (C3-C7)-Cycloalkyl substituiert sein können,
worin (C3-C7)-Cycloalkyl seinerseits mit 1 oder 2 Substituenten unabhängig voneinander ausgewählt aus der Gruppe (C1-C4)-Alkyl, Hydroxy, Oxo und (C1-C4)-Alkoxy substituiert sein kann,
und
wobei 5- oder 6-gliedriges Heterocyclyl mit 1 bis 3 Substituenten unabhängig voneinander ausgewählt aus der Gruppe Oxo, Thioxo, Hydroxy, (C₁-C₆)-Alkyl, (C₁-C₆)-Alkoxy, (C₁-C₆)-Alkylcarbonyl, Amino, Mono-(C₁-C₆)-alkylamino, Di-(C₁-C₆)-alkylamino und (C₃-C₇)-Cycloalkyl substituiert sein kann,
worin (C₁-C₆)-Alkyl mit 1 bis 3 Substituenten unabhängig voneinander ausgewählt aus der Gruppe Fluor, Oxo, Hydroxy, Trifluormethyl, (C₁-C₄)-Alkoxy, (C₁-C₄)-Alkylcarbonyloxy, Amino, Mono-(C₁-C₄)-alkylamino, Di-(C₁-C₄)-alkylamino und (C₃-C₇)-Cycloalkyl substituiert sein kann,
worin (C₃-C₇)-Cycloalkyl seinerseits mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
worin (C₁-C₆)-Alkylcarbonyl mit einem Substituenten ausgewählt aus der Gruppe Hydroxy und (C₁-C₄)-Alkoxy substituiert sein kann,
und
worin (C3-C7)-Cycloalkyl mit 1 oder 2 Substituenten unabhängig voneinander ausgewählt aus der Gruppe (C₁-C₄)-Alkyl, Hydroxy, Oxo und (C₁-C₄)-Alkoxy substituiert sein kann,
und
wobei Phenyl und 5- oder 6-gliedriges Heteroaryl mit 1 bis 3 Substituenten unabhängig voneinander ausgewählt aus der Gruppe Halogen, Cyano, Hydroxy, (C₁-C₆)-Alkyl, (C₁-C₆)-Alkoxy, (C₃-C₇)-Cycloalkoxy und -NR^{A}R^{B} substituiert sein können,
worin (C1-C6)-Alkyl mit 1 bis 3 Substituenten ausgewählt aus der Gruppe Fluor substituiert sein kann,
und
worin (C1-C6)-Alkoxy mit 1 bis 3 Substituenten unabhängig voneinander ausgewählt aus der Gruppe Fluor, Trifluormethyl, (C3-C7)-Cycloalkyl, Oxo, Hydroxy, (C1-C4)-Alkoxy, Hydroxycarbonyl, Amino, Mono-(C1-C4)-alkylamino und Di-(C1-C4)-alkylamino substituiert sein kann,
und
worin (C3-C7)-Cycloalkoxy mit 1 oder 2 Substituenten unabhängig voneinander ausgewählt aus der Gruppe (C1-C4)-Alkyl, Hydroxy, Oxo und (C1-C4)-Alkoxy substituiert sein können,
und
worin
R^{A} für Wasserstoff oder (C₁-C₆)-Alkyl steht,
worin (C₁-C₆)-Alkyl seinerseits mit einem Substituenten ausgewählt aus der Gruppe Hydroxy und (C₁-C₄)-Alkoxy substituiert sein kann,
R^{B} für Wasserstoff, (C₁-C₆)-Alkyl, (C₃-C₇)-Cycloalkyl, (C₁-C₄)-Alkylsulfonyl oder (C₃-C₇)-Cycloalkylsulfonyl steht,
worin (C₁-C₆)-Alkyl seinerseits mit 1 oder 2 Substituenten unabhängig voneinander ausgewählt aus der Gruppe (C3-C7)-Cycloalkyl, Oxo, Hydroxy, (C1-C4)-Alkoxy, Hydroxycarbonyl, Amino, Mono-(C1-C4)-alkylamino und Di-(C1-C4)-alkylamino substituiert sein kann,
und
worin (C3-C7)-Cycloalkyl seinerseits mit 1 oder 2 Substituenten unabhängig voneinander ausgewählt aus der Gruppe (C1-C4)-Alkyl, Hydroxy, Oxo und (C1-C4)-Alkoxy substituiert sein kann,
oder
worin zwei benachbarte Substituenten am Phenyl zusammen mit den Kohlenstoffatomen, an die sie gebunden sind, ein 1,3-Dioxolan oder 2,2-Difluor-1,3-dioxolan bilden können, sowie ihre N-Oxide, Salze, Solvate, Salze der N-Oxide und Solvate der N-Oxide.

2. Verbindung der Formel (I) nach Anspruch 1, in welcher der Ring Q für eine Gruppe der Formel 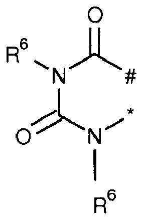 steht, wobei
* die Anknüpfstelle an das C2-Atom bedeutet,
# die Anknüpfstelle an das C3-Atom bedeutet,
R⁶ für jeweils Wasserstoff oder Methyl steht,
X für S oder O steht,
R¹ für Phenyl oder 5- oder 6-gliedriges Heteroaryl steht,
wobei Phenyl oder 5- oder 6-gliedriges Heteroaryl mit 1 oder 2 Substituenten unabhängig voneinander ausgewählt aus der Gruppe Fluor, Chlor, Cyano, (C₁-C₄)-Alkyl, Trifluormethyl, Hydroxy, (C₁-C₄)-Alkoxy, Amino, Hydroxycarbonyl, (C₁-C₄)-Alkoxycarbonyl, Aminocarbonyl, Phenyl und 5- oder 6-gliedriges Heteroaryl substituiert sind,
worin Phenyl und 5- oder 6-gliedriges Heteroaryl mit 1 bis 3 Substituenten unabhängig voneinander ausgewählt aus der Gruppe Fluor, Chlor, Nitro, Cyano, (C₁-C₄)-Alkyl, Difluormethyl, Trifluormethyl, Hydroxy, (C₁-C₄)-Alkoxy, Difluormethoxy, Trifluormethoxy, Amino, Hydroxycarbonyl und (C₁-C₄)-Alkoxycarbonyl substituiert sein können,
R² für Cyclohexyl, Tetrahydropyranyl, Piperidinyl, Piperazinyl, Morpholinyl, Phenyl, Pyrazolyl, Imidazolyl, Oxazolyl, Thiazolyl oder Pyridyl steht,
wobei Cyclohexyl mit einem Substituenten ausgewählt aus der Gruppe Hydroxy und (C₁-C₄)-Alkoxy substituiert sein kann,
worin (C₂-C₄)-Alkoxy mit 1 oder 2 Substituenten unabhängig voneinander ausgewählt aus der Gruppe Hydroxy und Methoxy substituiert sein kann,
und
wobei Piperidinyl, Piperazinyl und Morpholinyl mit einem Substituenten ausgewählt aus der Gruppe (C₁-C₄)-Alkyl, Hydroxy, (C₁-C₄)-Alkoxy und (C₁-C₄)-Alkylcarbonyl substituiert sein können,
worin (C₁-C₄)-Alkyl mit 1 oder 2 Substituenten unabhängig voneinander ausgewählt aus der Gruppe Hydroxy, Methoxy, Ethoxy, Methylcarbonyloxy und Ethylcarbonyloxy substituiert sein kann,
und
worin (C₁-C₄)-Alkylcarbonyl mit einem Substituenten ausgewählt aus der Gruppe Hydroxy, Methoxy und Ethoxy substituiert sein kann,
und
wobei Phenyl und Pyridyl mit 1 bis 3 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können,
worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
und
wobei Pyrazolyl, Imidazolyl, Oxazolyl und Thiazolyl mit 1 oder 2 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können,
worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
sowie ihre Salze, Solvate und Solvate der Salze.

3. Verbindung der Formel (I) nach Anspruch 1 oder 2, in welcher der Ring Q für eine Gruppe der Formel 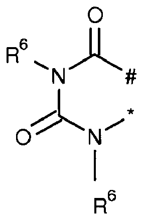 steht, wobei
* die Anknüpfstelle an das C2-Atom bedeutet,
# die Anknüpfstelle an das C3-Atom bedeutet,
R⁶ für jeweils Wasserstoff oder Methyl steht,
X für S oder O steht,
R¹ für Phenyl oder 5- oder 6-gliedriges Heteroaryl steht,
wobei Phenyl oder 5- oder 6-gliedriges Heteroaryl mit 1 oder 2 Substituenten unabhängig voneinander ausgewählt aus der Gruppe Fluor, Chlor, Cyano, Methyl, Ethyl, Trifluormethyl, Hydroxy, Methoxy, Ethoxy, Amino, Hydroxycarbonyl, Methoxycarbonyl, Ethoxycarbonyl, Aminocarbonyl, Phenyl und 5- oder 6-gliedriges Heteroaryl substituiert sind,
worin Phenyl und 5- oder 6-gliedriges Heteroaryl mit 1 bis 3 Substituenten unabhängig voneinander ausgewählt aus der Gruppe Fluor, Chlor, Methyl, Ethyl, Difluormethyl, Trifluormethyl, Hydroxy, Methoxy, Ethoxy, Amino, Hydroxycarbonyl, Methoxycarbonyl und Ethoxycarbonyl substituiert sein können,
R² für Phenyl, Pyrazolyl oder Pyridyl steht,
wobei Phenyl und Pyridyl mit 1 oder 2 Substituenten unabhängig voneinander ausgewählt aus der Gruppe Fluor, Chlor, Cyano, Hydroxy, (C₁-C₄)-Alkyl und (C₁-C₄)-Alkoxy substituiert sein können,
worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
und
wobei Pyrazolyl mit einem Substituenten ausgewählt aus der Gruppe Fluor, Chlor, Cyano, Hydroxy, (C₁-C₄)-Alkyl und (C₁-C₄)-Alkoxy substituiert sein kann,
worin (C₂-C₄)-Alkoxy mit 1 oder 2 Substituenten unabhängig voneinander ausgewählt aus der Gruppe Oxo, Hydroxy, (C₁-C₄)-Alkoxy, Hydroxycarbonyl und Amino substituiert sein kann,
sowie ihre Salze, Solvate und Solvate der Salze.

4. Verbindung der Formel (I) nach Anspruch 1, 2 oder 3, in welcher der Ring Q für eine Gruppe der Formel 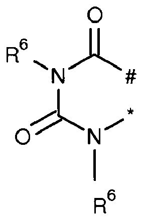 steht, worin
* die Anknüpfstelle an das C2-Atom bedeutet,
# die Anknüpfstelle an das C3-Atom bedeutet,
R⁶ für Wasserstoff steht,
X für S oder O steht,
R¹ für Thiazolyl oder Oxazolyl steht,
wobei Thiazolyl und Oxazolyl mit einem Substituenten Phenyl substituiert sind,
worin Phenyl mit einem Substituenten ausgewählt aus der Gruppe Fluor, Chlor, Cyano, Methyl, Methoxy, Hydroxycarbonyl und Methoxycarbonyl substituiert sein kann,
und
wobei Thiazolyl und Oxazolyl mit einem Substituenten ausgewählt aus der Gruppe Fluor, Chlor, Cyano, Methyl, Ethyl, Methoxy, Amino, Hydroxycarbonyl und Methoxycarbonyl substituiert sein können,
R² für eine Gruppe der Formel 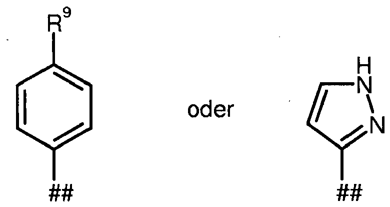
steht, wobei
## die Anknüpfstelle an den Bicyclus bedeutet,
worin
R⁹ für Wasserstoff oder (C₁-C₄)-Alkoxy steht,
worin (C₂-C₄)-Alkoxy mit 1 oder 2 Substituenten Hydroxy substituiert sein kann,
sowie ihre Salze, Solvate und Solvate der Salze.

5. Verfahren zur Herstellung von Verbindungen der Formel (I), wie in den Ansprüchen 1 bis 4 definiert, **dadurch gekennzeichnet, dass** man
[C] eine Verbindung der Formel (V) 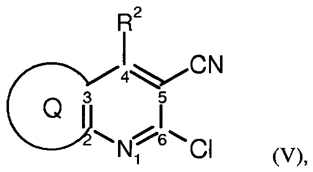 in welcher R² die in den Ansprüchen 1 bis 4 angegebene Bedeutung hat und der Ring Q für eine Gruppe der Formel 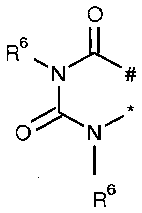 steht, wobei
* die Anknüpfstelle an das C2-Atom bedeutet,
# die Anknüpfstelle an das C3-Atom bedeutet,
R⁶ für Wasserstoff, (C₁-C₄)-Alkyl oder Allyl steht,
worin (C₁-C₄)-Alkyl mit einem Substituenten ausgewählt aus der Gruppe Hydroxycarbonyl, (C₁-C₄)-Alkoxycarbonyl und Amino substituiert sein kann,
in einem inerten Lösungsmittel zuerst mit einem Alkalisulfid, wie beispielsweise Natriumsulfid, zu einer Verbindung der Formel (VI) 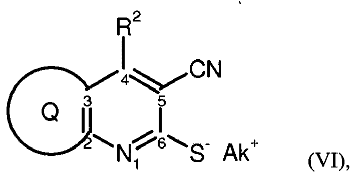 in welcher R² die zuvor angegebene Bedeutung hat,
Ak⁺ für ein Alkalisalz, vorzugsweise Natriumsalz, steht und
der Ring Q für eine Gruppe der Formel 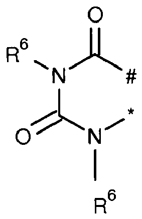 steht, wobei
* die Anknüpfstelle an das C2-Atom bedeutet,
# die Anknüpfstelle an das C3-Atom bedeutet,
R⁶ für Wasserstoff, (C₁-C₄)-Alkyl oder Allyl steht,
worin (C₁-C₄)-Alkyl mit einem Substituenten ausgewählt aus der Gruppe Hydroxycarbonyl, (C₁-C₄)-Alkoxycarbonyl und Amino substituiert sein kann,
umsetzt und diese anschliessend in Gegenwart einer geeigneten Base mit der Verbindung der Formel (VII) 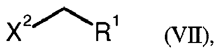 in welcher R¹ die in den Ansprüchen 1 bis 4 angegebene Bedeutung hat und
X² für eine geeignete Abgangsgruppe, vorzugsweise für Halogen, insbesondere Chlor, Brom oder Iod, oder für Mesylat, Tosylat oder Triflat steht,
zu einer Verbindung der Formel (I-C) 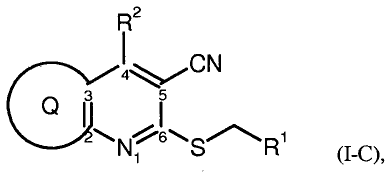 in welcher R¹ und R² jeweils die zuvor angegebenen Bedeutungen haben,
der Ring Q für eine Gruppe der Formel 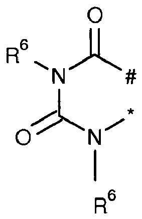 steht, wobei
* die Anknüpfstelle an das C2-Atom bedeutet,
# die Anknüpfstelle an das C3-Atom bedeutet,
R⁶ für Wasserstoff, (C₁-C₄)-Alkyl oder Allyl steht,
worin (C₁-C₄)-Alkyl mit einem Substituenten ausgewählt aus der Gruppe Hydroxycarbonyl, (C₁-C₄)-Alkoxycarbonyl und Amino substituiert sein kann,
umsetzt,
oder
[D] eine Verbindung der Formel (V) in einem inerten Lösungsmittel in Gegenwart einer Base mit einer Verbindung der Formel (VIII) 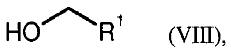 in welcher R¹ die in den Ansprüchen 1 bis 4 angegebene Bedeutung hat zu Verbindungen der Formel (I-D) 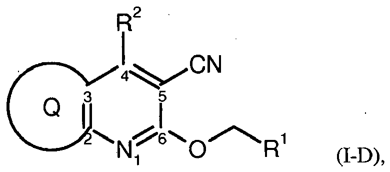 in welcher R¹ und R² jeweils die in den Ansprüchen bis 4 angegebenen Bedeutungen haben, und
der Ring Q für eine Gruppe der Formel 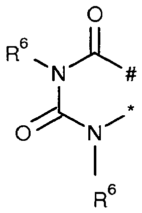 steht, wobei
* die Anknüpfstelle an das C2-Atom bedeutet,
# die Anknüpfstelle an das C3-Atom bedeutet,
R⁶ für jeweils Wasserstoff, (C₁-C₄)-Alkyl oder Allyl steht,
worin (C₁-C₄)-Alkyl mit einem Substituenten ausgewählt aus der Gruppe Hydroxycarbonyl, (C₁-C₄)-Alkoxycarbonyl und Amino substituiert sein kann,
umsetzt,
anschließend die resultierenden Verbindungen der Formeln (I-C) und (I-D) gegebenenfalls mit den entsprechenden (i) Lösungsmitteln und/oder (ii) Basen oder Säuren in ihre Solvate, Salze und/oder Solvate der Salze überführt.

6. Verbindung der Formel (I), wie in einem der Ansprüche 1 bis 4 definiert, zur Verwendung in der Behandlung und/oder Prävention von Krankheiten.

7. Verbindung gemäß Anspruch 6 zur Verwendung in einem Verfahren zur Behandlung und/oder Prophylaxe von Diabetes, Metabolischem Syndrom und Dyslipidämien.

8. Verwendung einer Verbindung der Formel (I), wie in einem der Ansprüche 1 bis 4 definiert, zur Herstellung eines Arzneimittels zur Behandlung und/oder Prävention von koronarer Herzerkrankung, akutem Koronarsyndrom, Angina pectoris, Herzinsuffizienz, Myokardinfarkt und Vorhofflimmern.

9. Verwendung gemäß Anspruch 8 zur Herstellung eines Arzneimittels zur Behandlung und/oder Prävention von koronarer Herzerkrankung, akutem Koronarsyndrom, Angina pectoris, Herzinsuffizienz, Myokardinfarkt, Vorhofflimmern und Hypertonie.

10. Verwendung einer Verbindung der Formel (I), wie in einem der Ansprüche 1 bis 4 definiert, zur Herstellung eines Arzneimittels zur Behandlung und/oder Prävention von Diabetes, Metabolischem Syndrom und Dyslipidämien.

11. Arzneimittel enthaltend eine Verbindung der Formel (I), wie in einem der Ansprüche 1 bis 4 definiert, in Kombination mit einem inerten, nicht-toxischen, pharmazeutisch geeigneten Hilfsstoff.

12. Arzneimittel enthaltend eine Verbindung der Formel (I), wie in einem der Ansprüche 1 bis 4 definiert, in Kombination mit einem oder mehreren weiteren Wirkstoffen ausgewählt aus der Gruppe bestehend aus den Fettstoffwechsel verändernden Wirkstoffen, Antidiabetika, blutdrucksenkenden Wirkstoffen und antithrombotisch wirkenden Mitteln.

13. Arzneimittel nach Anspruch 11 oder 12 zur Behandlung und/oder Prävention von koronarer Herzerkrankung, akutem Koronarsyndrom, Angina pectoris, Herzinsuffizienz, Myokardinfarkt, Vorhofflimmern und Hypertonie.

14. Arzneimittel nach Anspruch 11 oder 12 zur Behandlung und/oder Prävention von Diabetes, Metabolischem Syndrom und Dyslipidämien.

## Claims

1. Compound of the formula (I) 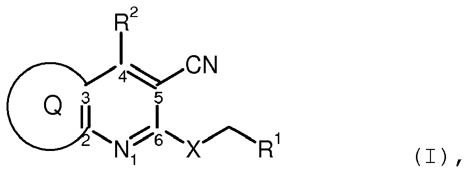 in which
ring Q represents a group of the formula 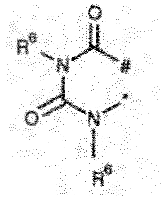 where
* represents the point of attachment to the C2 atom,
# represents the point of attachment to the C3 atom,
R⁶ represents in each case hydrogen, (C₁-C₄)-alkyl or allyl,
in which (C₁-C₄)-alkyl may be substituted by a substituent selected from the group consisting of hydroxycarbonyl, (C₁-C₄)-alkoxycarbonyl and amino,
X represents S or O,
R¹ represents (C₆-C₁₀)-aryl or 5- to 10-membered heteroaryl,
where (C₆-C₁₀)-aryl and 5- to 10-membered heteroaryl may be substituted by 1 or 2 substituents independently of one another selected from the group consisting of halogen, nitro, cyano, (C₁-C₆)-alkyl, trifluoromethyl, hydroxyl, (C₁-C₆)-alkoxy, amino, mono- (C₁-C₆)-alkylamino, di- (C₁-C₆)-alkylamino, hydroxycarbonyl, (C₁-C₆)-alkoxy-carbonyl, aminocarbonyl, mono-(C₁-C₆)-alkylaminocarbonyl, di-(C₁-C₆)-alkylaminocarbonyl, pyrrolidino, piperidino, morpholino, piperazino and N'-(C₁-C₄)-alkylpiperazino, phenyl and 5- or 6-membered heteroaryl,
in which phenyl and 5- or 6-membered heteroaryl may be substituted by 1 to 3 substituents independently of one another selected from the group consisting of halogen, nitro, cyano, (C₁-C₆)-alkyl, difluoromethyl, trifluoromethyl, hydroxyl, (C₁-C₆)-alkoxy, difluoromethoxy, trifluoromethoxy, amino, mono-(C₁-C₆)-alkylamino, di-(C₁-C₆)-alkylamino, hydroxycarbonyl and (C₁-C₆)-alkoxycarbonyl,
R² represents (C₅-C₆)-cycloalkyl, 5- or 6-membered heterocyclyl, phenyl or 5- or 6-membered heteroaryl,
where (C₅-C₆)-cycloalkyl may be substituted by 1 or 2 substituents independently of one another selected from the group consisting of (C₁-C₆)-alkyl, hydroxyl, oxo, (C₁-C₆)-alkoxy, amino, mono- (C₁-C₆)-alkylamino and di- (C₁-C₆)-alkylamino,
in which (C₁-C₆)-alkyl and (C₁-C₆)-alkoxy may be substituted by 1 or 2 substituents independently of one another selected from the group consisting of hydroxyl, (C₁-C₄)-alkoxy and (C₃-C₇)-cycloalkyl,
in which (C₃-C₇)-cycloalkyl for its part may be substituted by 1 or 2 substituents independently of one another selected from the group consisting of (C₁-C₄)-alkyl, hydroxyl, oxo and (C₁-C₄)-alkoxy,
and
where 5- or 6-membered heterocyclyl may be substituted by 1 to 3 substituents independently of one another selected from the group consisting of oxo, thioxo, hydroxyl, (C₁-C₆)-alkyl, (C₁-C₆)-alkoxy, (C₁-C₆)-alkylcarbonyl, amino, mono-(C₁-C₆)-alkylamino, di-(C₁-C₆)-alkylamino and (C₃-C₇)-cycloalkyl,
in which (C₁-C₆)-alkyl may be substituted by 1 to 3 substituents independently of one another selected from the group consisting of fluorine, oxo, hydroxyl, trifluoromethyl, (C₁-C₄)-alkoxy, (C₁-C₄)-alkylcarbonyloxy, amino, mono-(C₁-C₄)-alkylamino, di-(C₁-C₄)-alkylamino and (C₃-C₇)-cycloalkyl,
in which (C₃-C₇)-cycloalkyl for its part may be substituted by 1 or 2 substituents independently of one another selected from the group consisting of (C₁-C₄)-alkyl, hydroxyl, oxo and (C₁-C₄)-alkoxy,
and
in which (C₁-C₆)-alkylcarbonyl may be substituted by a substituent selected from the group consisting of hydroxyl and (C₁-C₄)-alkoxy,
and
in which (C₃-C₇)-cycloalkyl may be substituted by 1 or 2 substituents independently of one another selected from the group consisting of (C₁-C₄)-alkyl, hydroxyl, oxo and (C₁-C₄)-alkoxy,
and
where phenyl and 5- or 6-membered heteroaryl may be substituted by 1 to 3 substituents independently of one another selected from the group consisting of halogen, cyano, hydroxyl, (C₁-C₆)-alkyl, (C₁-C₆)-alkoxy, (C₃-C₇)-cycloalkoxy and -NR^{A}R^{B},
in which (C₁-C₆)-alkyl may be substituted by 1 to 3 substituents selected from the group consisting of fluorine,
and
in which (C₁-C₆)-alkoxy may be substituted by 1 to 3 substituents independently of one another selected from the group consisting of fluorine, trifluoromethyl, (C₃-C₇)-cycloalkyl, oxo, hydroxyl, (C₁-C₄)-alkoxy, hydroxycarbonyl, amino, mono-(C₁-C₄)-alkylamino and di-(C₁-C₄)-alkylamino,
and
in which (C₃-C₇)-cycloalkoxy may be substituted by 1 or 2 substituents independently of one another selected from the group consisting of (C₁-C₄)-alkyl, hydroxyl, oxo and (C₁-C₄)-alkoxy,
and
in which
R^{A} represents hydrogen or (C₁-C₆)-alkyl,
in which (C₁-C₆)-alkyl for its part may be substituted by a substituent selected from the group consisting of hydroxyl and (C₁-C₄)-alkoxy,
R^{B} represents hydrogen, (C₁-C₆)-alkyl, (C₃-C₇)-cycloalkyl, (C₁-C₄)-alkylsulfonyl or (C₃-C₇)-cycloalkylsulfonyl,
in which (C₁-C₆)-alkyl for its part may be substituted by 1 or 2 substituents independently of one another selected from the group consisting of (C₃-C₇)-cycloalkyl, oxo, hydroxyl, (C₁-C₄)-alkoxy, hydroxycarbonyl, amino, mono-(C₁-C₄) -alkylamino and di-(C₁-C₄)-alkylamino,
and
in which (C₃-C₇)-cycloalkyl for its part may be substituted by 1 or 2 substituents independently of one another selected from the group consisting of (C₁-C₄)-alkyl, hydroxyl, oxo and (C₁-C₄)-alkoxy,
or
in which two adjacent substituents at the phenyl together with the carbon atoms to which they are attached may form a 1,3-dioxolane or 2,2-difluoro-1,3-dioxolane,
or an N-oxide, salt, solvate, salt of the N-oxide or solvate of the N-oxide.

2. Compound of the formula (I) according to Claim 1 in which ring Q represents a group of the formula 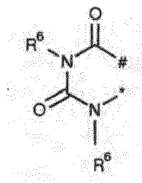 where
* represents the point of attachment to the C2 atom,
# represents the point of attachment to the C3 atom,
R⁶ represents in each case hydrogen or methyl,
X represents S or O,
R¹ represents phenyl or 5- or 6-membered heteroaryl,
where phenyl and 5- or 6-membered heteroaryl are substituted by 1 or 2 substituents independently of one another selected from the group consisting of fluorine, chlorine, cyano, (C₁-C₄)-alkyl, trifluoromethyl, hydroxyl, (C₁-C₄)-alkoxy, amino, hydroxycarbonyl, (C₁-C₄)-alkoxycarbonyl, aminocarbonyl, phenyl and 5- or 6-membered heteroaryl,
in which phenyl and 5- or 6-membered heteroaryl may be substituted by 1 to 3 substituents independently of one another selected from the group consisting of fluorine, chlorine, nitro, cyano, (C₁-C₄)-alkyl, difluoromethyl, trifluoromethyl, hydroxyl, (C₁-C₄)-alkoxy, difluoromethoxy, trifluoromethoxy, amino, hydroxycarbonyl and (C₁-C₄)-alkoxycarbonyl,
R² represents cyclohexyl, tetrahydropyranyl, piperidinyl, piperazinyl, morpholinyl, phenyl, pyrazolyl, imidazolyl, oxazolyl, thiazolyl or pyridyl,
where cyclohexyl may be substituted by a substituent selected from the group consisting of hydroxyl and (C₁-C₄)-alkoxy,
in which (C₂-C₄)-alkoxy may be substituted by 1 or 2 substituents independently of one another selected from the group consisting of hydroxyl and methoxy,
and
where piperidinyl, piperazinyl and morpholinyl may be substituted by a substituent selected from the group consisting of (C₁-C₄)-alkyl, hydroxyl, (C₁-C₄)-alkoxy and (C₁-C₄)-alkylcarbonyl,
in which (C₁-C₄)-alkyl may be substituted by 1 or 2 substituents independently of one another selected from the group consisting of hydroxyl, methoxy, ethoxy, methylcarbonyloxy and ethylcarbonyloxy,
and
in which (C₁-C₄)-alkylcarbonyl may be substituted by a substituent selected from the group consisting of hydroxyl, methoxy and ethoxy,
and
where phenyl and pyridyl may be substituted by 1 to 3 substituents independently of one another selected from the group consisting of fluorine, chlorine, cyano, hydroxyl, (C₁-C₄)-alkyl and (C₁-C₄)-alkoxy,
in which (C₂-C₄)-alkoxy may be substituted by 1 or 2 substituents independently of one another selected from the group consisting of oxo, hydroxyl, (C₁-C₄)-alkoxy, hydroxycarbonyl and amino,
and
where pyrazolyl, imidazolyl, oxazolyl and thiazolyl may be substituted by 1 or 2 substituents independently of one another selected from the group consisting of fluorine, chlorine, cyano, hydroxyl, (C₁-C₄)-alkyl and (C₁-C₄)-alkoxy,
in which (C₂-C₄)-alkoxy may be substituted by 1 or 2 substituents independently of one another selected from the group consisting of oxo, hydroxyl, (C₁-C₄)-alkoxy, hydroxycarbonyl and amino,
or a salt, solvate or solvate of a salt thereof.

3. Compound of the formula (I) according to Claim 1 or 2 in which ring Q represents a group of the formula 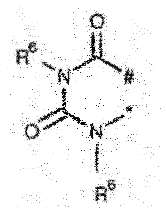 where
* represents the point of attachment to the C2 atom,
# represents the point of attachment to the C3 atom,
R⁶ represents in each case hydrogen or methyl,
X represents S or O,
R¹ represents phenyl or 5- or 6-membered heteroaryl,
where phenyl and 5- or 6-membered heteroaryl are substituted by 1 or 2 substituents independently of one another selected from the group consisting of fluorine, chlorine, cyano, methyl, ethyl, trifluoromethyl, hydroxyl, methoxy, ethoxy, amino, hydroxycarbonyl, methoxycarbonyl, ethoxycarbonyl, aminocarbonyl, phenyl and 5-or 6-membered heteroaryl,
in which phenyl and 5- or 6-membered heteroaryl may be substituted by 1 to 3 substituents independently of one another selected from the group consisting of fluorine, chlorine, methyl, ethyl, difluoromethyl, trifluoromethyl, hydroxyl, methoxy, ethoxy, amino, hydroxycarbonyl, methoxycarbonyl and ethoxycarbonyl,
R² represents phenyl, pyrazolyl or pyridyl,
where phenyl and pyridyl may be substituted by 1 or 2 substituents independently of one another selected from the group consisting of fluorine, chlorine, cyano, hydroxyl, (C₁-C₄)-alkyl and (C₁-C₄)-alkoxy,
in which (C₂-C₄)-alkoxy may be substituted by 1 or 2 substituents independently of one another selected from the group consisting of oxo, hydroxyl, (C₁-C₄)-alkoxy, hydroxycarbonyl and amino,
and
where pyrazolyl may be substituted by a substituent selected from the group consisting of fluorine, chlorine, cyano, hydroxyl, (C₁-C₄)-alkyl and (C₁-C₄)-alkoxy,
in which (C₂-C₄)-alkoxy may be substituted by 1 or 2 substituents independently of one another selected from the group consisting of oxo, hydroxyl, (C₁-C₄)-alkoxy, hydroxycarbonyl and amino,
or a salt, solvate or solvate of a salt thereof.

4. Compound of the formula (I) according to Claim 1, 2 or 3 in which ring Q represents a group of the formula 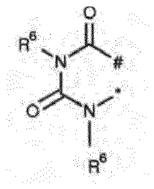 in which
* represents the point of attachment to the C2 atom,
# represents the point of attachment to the C3 atom,
R⁶ represents hydrogen,
X represents S or O,
R¹ represents thiazolyl or oxazolyl,
where thiazolyl and oxazolyl are substituted by a phenyl substituent,
in which phenyl may be substituted by a substituent selected from the group consisting of fluorine, chlorine, cyano, methyl, methoxy, hydroxycarbonyl and methoxycarbonyl,
and
where thiazolyl and oxazolyl may be substituted by a substituent selected from the group consisting of fluorine, chlorine, cyano, methyl, ethyl, methoxy, amino, hydroxycarbonyl and methoxycarbonyl,
R² represents a group of the formula 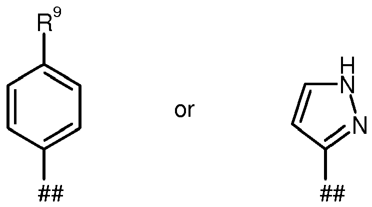 where
## represents the point of attachment to the bicycle,
in which
R⁹ represents hydrogen or (C₁-C₄)-alkoxy,
in which (C₂-C₄)-alkoxy may be substituted by 1 or 2 hydroxyl substituents,
or a salt, solvate or solvate of a salt thereof.

5. Process for preparing compounds of the formula (I) as defined in any of Claims 1 to 4, **characterized in that**
[C] a compound of the formula (V) 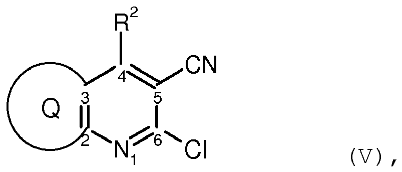 in which R² has the meaning given in any of Claims 1 to 4 and
ring Q represents a group of the formula 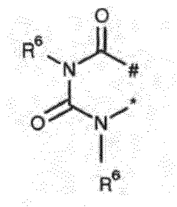 where
* represents the point of attachment to the C2 atom,
# represents the point of attachment to the C3 atom,
R⁶ represents hydrogen, (C₁-C₄)-alkyl or allyl,
in which (C₁-C₄)-alkyl may be substituted by a substituent selected from the group consisting of hydroxycarbonyl, (C₁-C₄)-alkoxycarbonyl and amino,
is reacted in an inert solvent initially with an alkali metal sulfide, such as, for example, sodium sulfide, to give a compound of the formula (VI) 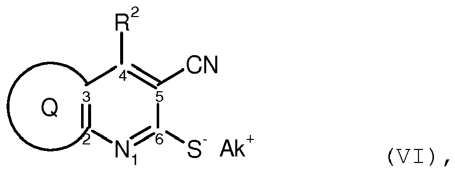 in which R² has the meaning given above,
Ak⁺ represents an alkali metal salt, preferably a sodium salt,
and
ring Q represents a group of the formula 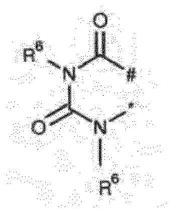 where
* represents the point of attachment to the C2 atom,
# represents the point of attachment to the C3 atom,
R⁶ represents in each case hydrogen, (C₁-C₄)-alkyl or allyl,
in which (C₁-C₄)-alkyl may be substituted by a substituent selected from the group consisting of hydroxycarbonyl, (C₁-C₄)-alkoxycarbonyl and amino,
and this is then reacted in the presence of a suitable base with the compound of the formula (VII) 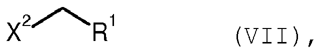 in which R¹ has the meaning given in any of Claims 1 to 4 and
X² represents a suitable leaving group, preferably halogen, in particular chlorine, bromine or iodine, or represents mesylate, tosylate or triflate,
to give a compound of the formula (I-C) 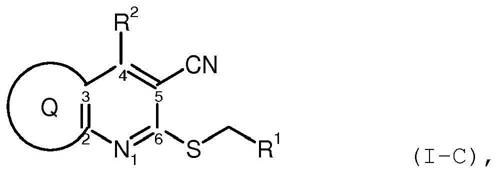 in which R¹ and R² each have the meanings given above,
ring Q represents a group of the formula 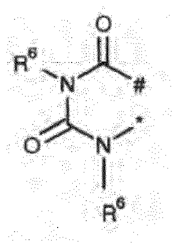 where
* represents the point of attachment to the C2 atom,
# represents the point of attachment to the C3 atom,
R⁶ represents hydrogen, (C₁-C₄)-alkyl or allyl,
in which (C₁-C₄)-alkyl may be substituted by a substituent selected from the group consisting of hydroxycarbonyl, (C₁-C₄)-alkoxycarbonyl and amino,
[D] a compound of the formula (V) is reacted in an inert solvent in the presence of a base with a compound of the formula (VIII) 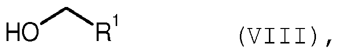 in which R¹ has the meaning given in any of Claims 1 to 4
to give compounds of the formula (I-D) 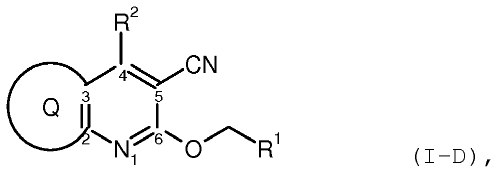 in which R¹ and R² each have the meanings given in any of Claims 1 to 4,
and
ring Q represents a group of the formula 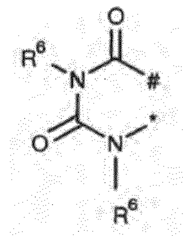 where
* represents the point of attachment to the C2 atom,
# represents the point of attachment to the C3 atom,
R⁶ represents in each case hydrogen, (C₁-C₄)-alkyl or allyl,
in which (C₁-C₄)-alkyl may be substituted by a substituent selected from the group consisting of hydroxycarbonyl, (C₁-C₄)-alkoxycarbonyl and amino,
the resulting compounds of the formulae (I-C) and (I-D) are then, if appropriate, converted with the appropriate (i) solvents and/or (ii) bases or acids into their solvates, salts and/or solvates of the salts.

6. Compound of the formula (I) as defined in any of Claims 1 to 4 for use in the treatment and/or prevention of diseases.

7. Compound according to Claim 6 for use in a method for the treatment and/or prophylaxis of diabetes, metabolic syndrome and dyslipidemias.

8. Use of a compound of the formula (I) as defined in any of Claims 1 to 4 for preparing a medicament for the treatment and/or prevention of coronary heart disease, acute coronary syndrome, angina pectoris, heart failure, myocardial infarction and atrial fibrillation.

9. Use according to Claim 8 for preparing a medicament for the treatment and/or prevention of coronary heart disease, acute coronary syndrome, angina pectoris, heart failure, myocardial infarction, atrial fibrillation and hypertension.

10. Use of a compound of the formula (I) as defined in any of Claims 1 to 4 for preparing a medicament for the treatment and/or prevention of diabetes, metabolic syndrome and dyslipidemias.

11. Medicament comprising a compound of the formula (I) as defined in any of Claims 1 to 4 in combination with an inert nontoxic pharmaceutically suitable auxiliary.

12. Medicament comprising a compound of the formula (I) as defined in any of Claims 1 to 4 in combination with one or more further active ingredients selected from the group consisting of lipid metabolism-altering active ingredients, antidiabetics, antihypertensive drugs and antithrombotic drugs.

13. Medicament according to Claim 11 or 12 for the treatment and/or prevention of coronary heart disease, acute coronary syndrome, angina pectoris, heart failure, myocardial infarction, atrial fibrillation and hypertension.

14. Medicament according to Claim 11 or 12 for the treatment and/or prevention of diabetes, metabolic syndrome and dyslipidemias.

## Revendications

1. Composé de formule (I), 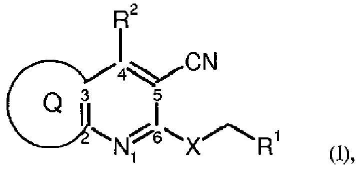 dans laquelle
le cycle Q représente un groupe de formule 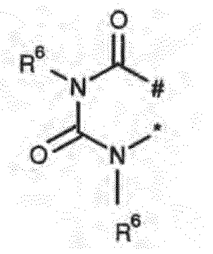 où
* signifie le site de liaison à l'atome C2,
# signifie le site de liaison à l'atome C3,
R⁶ représente à chaque fois hydrogène ou (C₁-C₄)-alkyle ou allyle,
(C₁-C₄)-alkyle pouvant être substitué par un substituant choisi dans le groupe formé par hydroxycarbonyle, (C₁-C₄)-alcoxycarbonyle et amino,
X représente S ou O,
R¹ représente (C₆-C₁₀)-aryle ou hétéroaryle de 5 à 10 chaînons, (C₆-C₁₀)-aryle et hétéroaryle de 5 à 10 chaînons pouvant être substitué par 1 ou 2 substituants choisis, indépendamment l'un de l'autre, dans le groupe formé par halogène, nitro, cyano, (C₁-C₆)-alkyle, trifluorométhyle, hydroxy, (C₁-C₆)-alcoxy, amino, mono-(C₁-C₆)-alkylamino, di-(C₁-C₆)-alkylamino, hydroxycarbonyle, (C₁-C₆)-alcoxycarbonyle, aminocarbonyle, mono-(C₁-C₆)-alkylaminocarbonyle, di-(C₁-C₆)-alkylaminocarbonyle, pyrrolidino, pipéridino, morpholino, pipérazino et N'-(C₁-C₄)-alkylpipérazino, phényle et hétéroaryle de 5 ou 6 chaînons,
phényle et hétéroaryle de 5 ou 6 chaînons pouvant être substitués par 1 à 3 substituants choisis, indépendamment les uns des autres, dans le groupe formé par halogène, nitro, cyano, (C₁-C₆)-alkyle, difluorométhyle, trifluorométhyle, hydroxy, (C₁-C₆)-alcoxy, difluorométhoxy, trifluorométhoxy, amino, mono-(C₁-C₆)-alkylamino, di-(C₁-C₆)-alkylamino, hydroxycarbonyle et (C₁-C₆)-alcoxycarbonyle,
R² représente (C₅-C₆)-cycloalkyle, hétérocyclyle de 5 ou 6 chaînons, phényle ou hétéroaryle de 5 ou 6 chaînons,
(C₅-C₆)-cycloalkyle pouvant être substitué par 1 ou 2 substituants choisis, indépendamment l'un de l'autre, dans le groupe formé par (C₁-C₆)-alkyle, hydroxy, oxo, (C₁-C₆)-alcoxy, amino, mono-(C₁-C₆)-alkylamino et di-(C₁-C₆)-alkylamino,
(C₁-C₆)-alkyle et (C₁-C₆)-alcoxy pouvant être substitués par 1 ou 2 substituants choisis, indépendamment l'un de l'autre, dans le groupe formé par hydroxy, (C₁-C₄)-alcoxy et (C₃-C₇)-cycloalkyle,
(C₃-C₇)-cycloalkyle pouvant à son tour être substitué par 1 ou 2 substituants choisis, indépendamment l'un de l'autre, dans le groupe formé par (C₁-C₄)-alkyle, hydroxy, oxo et (C₁-C₄)-alcoxy,
et
hétérocyclyle de 5 ou 6 chaînons pouvant être substitué par 1 à 3 substituants choisis, indépendamment les uns des autres, dans le groupe formé par oxo, thioxo, hydroxy, (C₁-C₆)-alkyle, (C₁-C₆)-alcoxy, (C₁-C₆)-alkylcarbonyle, amino, mono-(C₁-C₆)-alkylamino, di-(C₁-C₆)-alkylamino et (C₃-C₇)-cycloalkyle,
(C₁-C₆)-alkyle pouvant être substitué par 1 à 3 substituants choisis, indépendamment les uns des autres, dans le groupe formé par fluor, oxo, hydroxy, trifluorométhyle, (C₁-C₄)-alcoxy, (C₁-C₄)-alkylcarbonyloxy, amino, mono-(C₁-C₄)-alkylamino, di-(C₁-C₄)-alkylamino et (C₃-C₇)-cycloalkyle,
(C₃-C₇)-cycloalkyle pouvant à son tour être substitué par 1 ou 2 substituants choisis, indépendamment l'un de l'autre, dans le groupe formé par (C₁-C₄)-alkyle, hydroxy, oxo et (C₁-C₄)-alcoxy,
et
(C₁-C₆)-alkylcarbonyle pouvant être substitué par un substituant choisi dans le groupe formé par hydroxy et (C₁-C₄)-alcoxy, et (C₃-C₇)-cycloalkyle pouvant être substitué par 1 ou 2 substituants choisis, indépendamment l'un de l'autre, dans le groupe formé par (C₁-C₄)-alkyle, hydroxy, oxo et (C₁-C₄)-alcoxy,
et
phényle et hétéroaryle de 5 ou 6 chaînons pouvant être substitués par 1 à 3 substituants choisis, indépendamment les uns des autres, dans le groupe formé par halogène, cyano, hydroxy, (C₁-C₆)-alkyle, (C₁-C₆)-alcoxy, (C₃-C₇)-cycloalcoxy et -NR^{A}R^{B},
(C₁-C₆)-alkyle pouvant être substitué par 1 à 3 substituants choisis dans le groupe formé par fluor, et
(C₁-C₆)-alcoxy pouvant être substitué par 1 à 3 substituants choisis, indépendamment les uns des autres, dans le groupe formé par fluor, trifluorométhyle, (C₃-C₇)-cycloalkyle, oxo, hydroxy, (C₁-C₄)-alcoxy, hydroxycarbonyle, amino, mono-(C₁-C₄)-alkylamino et di-(C₁-C₄)-alkylamino, et (C₃-C₇)-cycloalcoxy pouvant être substitué par 1 ou 2 substituants choisis, indépendamment l'un de l'autre, dans le groupe formé par (C₁-C₄)-alkyle, hydroxy, oxo et (C₁-C₄)-alcoxy, et
où
R^{A} représente hydrogène ou (C₁-C₆)-alkyle, (C₁-C₆)-alkyle pouvant à son tour être substitué par un substituant choisi dans le groupe formé par hydroxy et (C₁-C₄)-alcoxy,
R^{B} représente hydrogène, (C₁-C₆)-alkyle, (C₃-C₇)-cycloalkyle, (C₁-C₄)-alkylsulfonyle ou (C₃-C₇)-cycloalkylsulfonyle,
(C₁-C₆)-alkyle pouvant à son tour être substitué par 1 ou 2 substituants choisis, indépendamment l'un de l'autre, dans le groupe formé par (C₃-C₇)-cycloalkyle, oxo, hydroxy, (C₁-C₄)-alcoxy, hydroxycarbonyle, amino, mono-(C₁-C₄)-alkylamino et di-(C₁-C₄)-alkylamino, et
(C₃-C₇)-cycloalkyle pouvant à son tour être substitué par 1 ou 2 substituants choisis, indépendamment l'un de l'autre, dans le groupe formé par (C₁-C₄)-alkyle, hydroxy, oxo et (C₁-C₄)-alcoxy,
ou
deux substituants adjacents sur phényle pouvant former ensemble avec les atomes de carbone auxquels ils sont liés un 1,3-dioxolane ou un 2,2-difluoro-1,3-dioxolane,
ainsi que ses N-oxydes, sels, solvates, sels des N-oxydes et solvates des N-oxydes.

2. Composé de formule (I) selon la revendication 1, le cycle Q représentant un groupe de formule 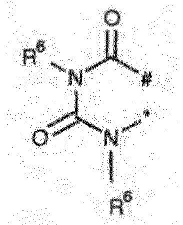 où
* signifie le site de liaison à l'atome C2,
# signifie le site de liaison à l'atome C3,
R⁶ représente hydrogène ou méthyle,
X représente S ou O,
R¹ représente phényle ou hétéroaryle de 5 ou 6 chaînons,
phényle ou hétéroaryle de 5 ou 6 chaînons étant substitué par 1 ou 2 substituants choisis, indépendamment l'un de l'autre, dans le groupe formé par fluor, chlore, cyano, (C₁-C₄)-alkyle, trifluorométhyle, hydroxy, (C₁-C₄)-alcoxy, amino, hydroxycarbonyle, (C₁-C₄)-alcoxycarbonyle, aminocarbonyle, phényle et hétéroaryle de 5 ou 6 chaînons,
phényle et hétéroaryle de 5 ou 6 chaînons pouvant être substitués par 1 à 3 substituants choisis, indépendamment les uns des autres, dans le groupe formé par fluor, chlore, nitro, cyano, (C₁-C₄)-alkyle, difluorométhyle, trifluorométhyle, hydroxy, (C₁-C₄)-alcoxy, difluorométhoxy, trifluorométhoxy, amino, hydroxycarbonyle et (C₁-C₄)-alcoxycarbonyle,
R² représente cyclohexyle, tétrahydropyrannyle, pipéridinyle, pipérazinyle, morpholinyle, phényle, pyrazolyle, imidazolyle, oxazolyle, thiazolyle ou pyridyle,
cyclohexyle pouvant être substitué par un substituant choisi dans le groupe formé par hydroxy et (C₁-C₄)-alcoxy,
(C₂-C₄)-alcoxy pouvant être substitué par 1 ou 2 substituants choisis, indépendamment l'un de l'autre, dans le groupe formé par hydroxy et méthoxy, et
pipéridinyle, pipérazinyle et morpholinyle pouvant être substitués par un substituant choisi dans le groupe formé par (C₁-C₄)-alkyle, hydroxy, (C₁-C₄)-alcoxy et (C₁-C₄)-alkylcarbonyle,
(C₁-C₄)-alkyle pouvant être substitué par 1 ou 2 substituants choisis, indépendamment l'un de l'autre, dans le groupe formé par hydroxy, méthoxy, éthoxy, méthylcarbonyloxy et éthylcarbonyloxy, et
(C₁-C₄)-alkylcarbonyle pouvant être substitué par un substituant choisi dans le groupe formé par hydroxy, méthoxy et éthoxy, et
phényle et pyridyle pouvant être substitués par 1 à 3 substituants choisis, indépendamment les uns des autres, dans le groupe formé par fluor, chlore, cyano, hydroxy, (C₁-C₄)-alkyle et (C₁-C₄)-alcoxy,
(C₂-C₄)-alcoxy pouvant être substitué par 1 ou 2 substituants choisis, indépendamment l'un de l'autre, dans le groupe formé par oxo, hydroxy, (C₁-C₄)-alcoxy, hydroxycarbonyle et amino, et
pyrazolyle, imidazolyle, oxazolyle et thiazolyle pouvant être substitués par 1 ou 2 substituants choisis, indépendamment l'un de l'autre, dans le groupe formé par fluor, chlore, cyano, hydroxy, (C₁-C₄)-alkyle et (C₁-C₄)-alcoxy,
(C₂-C₄)-alcoxy pouvant être substitué par 1 ou 2 substituants choisis, indépendamment l'un de l'autre, dans le groupe formé par oxo, hydroxy, (C₁-C₄)-alcoxy, hydroxycarbonyle et amino,
ainsi que ses sels, solvates et les solvates des sels.

3. Composé de formule (I) selon la revendication 1 ou 2, le cycle Q représentant un groupe de formule 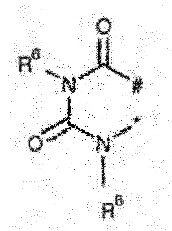 où
* signifie le site de liaison à l'atome C2,
# signifie le site de liaison à l'atome C3,
R⁶ représente à chaque fois hydrogène ou méthyle,
X représente S ou O,
R¹ représente phényle ou hétéroaryle de 5 ou 6 chaînons,
phényle ou hétéroaryle de 5 ou 6 chaînons étant substitué par 1 ou 2 substituants choisis, indépendamment l'un de l'autre, dans le groupe formé par fluor, chlore, cyano, méthyle, éthyle trifluorométhyle, hydroxy, méthoxy, éthoxy, amino, hydroxycarbonyle, méthoxycarbonyle, éthoxycarbonyle, aminocarbonyle, phényle et hétéroaryle de 5 ou 6 chaînons,
phényle et hétéroaryle de 5 ou 6 chaînons pouvant être substitués par 1 à 3 substituants choisis, indépendamment l'un de l'autre, dans le groupe formé par fluor, chlore, méthyle, éthyle, difluorométhyle, trifluorométhyle, hydroxy, méthoxy, éthoxy, amino, hydroxycarbonyle, méthoxycarbonyle et éthoxycarbonyle,
R² représente phényle, pyrazolyle ou pyridyle, phényle et pyridyle pouvant être substitués par 1 ou 2 substituants choisis, indépendamment l'un de l'autre, dans le groupe formé par fluor, chlore, cyano, hydroxy, (C₁-C₄)-alkyle et (C₁-C₄)-alcoxy,
(C₂-C₄)-alcoxy pouvant être substitué par 1 ou 2 substituants choisis, indépendamment l'un de l'autre, dans le groupe formé par oxo, hydroxy, (C₁-C₄)-alcoxy, hydroxycarbonyle et amino, et
pyrazolyle pouvant être substitué par un substituant choisi dans le groupe formé par fluor, chlore, cyano, hydroxy, (C₁-C₄)-alkyle et (C₁-C₄)-alcoxy,
(C₂-C₄)-alcoxy pouvant être substitué par 1 ou 2 substituants choisis, indépendamment l'un de l'autre, dans le groupe formé par oxo, hydroxy, (C₁-C₄)-alcoxy, hydroxycarbonyle et amino,
ainsi que ses sels, solvates et les solvates des sels.

4. Composé de formule (I) selon la revendication 1, 2 ou 3, le cycle Q représentant un groupe de formule 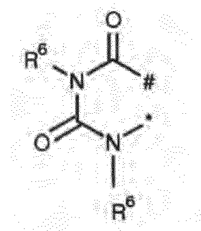 dans laquelle
* signifie le site de liaison à l'atome C2,
# signifie le site de liaison à l'atome C3,
R⁶ représente hydrogène,
X représente S ou O,
R¹ représente thiazolyle ou oxazolyle,
thiazolyle et oxazolyle étant substitués par un substituant phényle,
phényle pouvant être substitué par un substituant choisi dans le groupe fluor, chlore, cyano, méthyle, méthoxy, hydroxycarbonyle et méthoxycarbonyle, et
thiazolyle et oxazolyle pouvant être substitués par un substituant choisi dans le groupe fluor, chlore, cyano, méthyle, éthyle, méthoxy, amino, hydroxycarbonyle et méthoxycarbonyle,
R² représente un groupe de formule 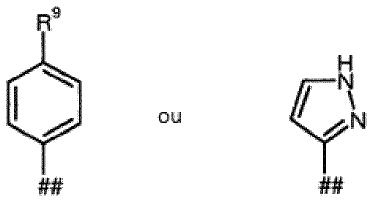 où
## signifie le site de liaison au bicycle,
où
R⁹ représente hydrogène ou (C₁-C₄)-alcoxy, (C₂-C₄)-alcoxy pouvant être substitué par 1 ou 2 substituants hydroxy,
ainsi que ses sels, solvates et les solvates des sels.

5. Procédé pour la préparation de composés de formule (I), tels que définis dans les revendications 1 à 4, **caractérisé en ce qu'**on
[C] transforme un composé de formule (V) 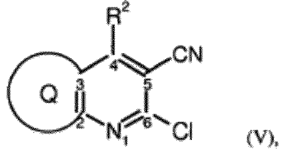 dans laquelle R² présente la signification indiquée dans les revendications 1 à 4 et
le cycle Q représente un groupe de formule 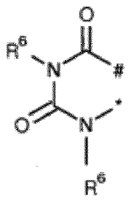
* signifiant le site de liaison à l'atome C2
# signifiant le site de liaison à l'atome C3
R⁶ représentant hydrogène, (C₁-C₄)-alkyle ou allyle
(C₁-C₄)-alkyle pouvant être substitué par un substituant choisi dans le groupe formé par hydroxycarbonyle, (C₁-C₄)-alcoxycarbonyle et amino
dans un solvant inerte, d'abord avec un sulfure de métal alcalin, tel que par exemple le sulfure de sodium, en un composé de formule (VI) 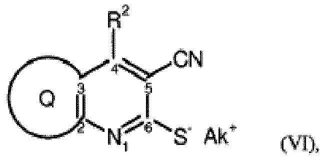 dans laquelle R² présente la signification indiquée ci-dessus,
Ak⁺ représente un sel de métal alcalin, de préférence un sel de sodium
et
le cycle Q représente un groupe de formule 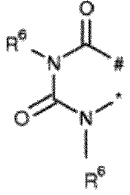
* signifiant le site de liaison à l'atome C2
# signifiant le site de liaison à l'atome C3
R⁶ représentant hydrogène, (C₁-C₄)-alkyle ou allyle
(C₁-C₄)-alkyle pouvant être substitué par un substituant choisi dans le groupe formé par hydroxycarbonyle, (C₁-C₄)-alcoxycarbonyle et amino
et on transforme ensuite celui-ci en présence d'une base appropriée avec le composé de formule (VII) 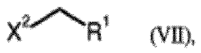 dans laquelle R¹ présente la signification indiquée dans les revendications 1 à 4 et
X² représente un groupe partant approprié, de préférence halogène, en particulier chlore, brome ou iode, ou mésylate, tosylate ou triflate,
en un composé de formule (I-C) 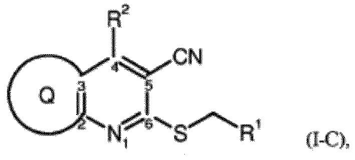 dans laquelle R¹ et R² présentent à chaque fois les significations indiquées ci-dessus, et
le cycle Q représente un groupe de formule 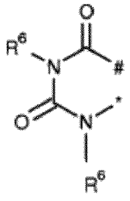
* signifiant le site de liaison à l'atome C2
# signifiant le site de liaison à l'atome C3
R⁶ représentant hydrogène, (C₁-C₄)-alkyle ou allyle
(C₁-C₄)-alkyle pouvant être substitué par un substituant choisi dans le groupe formé par hydroxycarbonyle, (C₁-C₄)-alcoxycarbonyle et amino
ou
[D] transforme un composé de formule (V) dans un solvant inerte en présence d'une base avec un composé de formule (VIII) 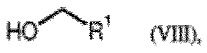 dans laquelle R¹ présente la signification indiquée dans les revendications 1 à 4,
en composés de formule (I-D) 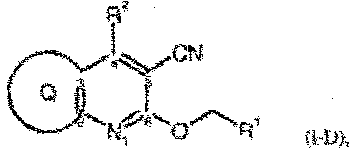 dans laquelle R¹ et R² présentent à chaque fois les significations indiquées dans les revendications 1 à 4, et
le cycle Q représente un groupe de formule 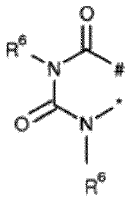
* signifiant le site de liaison à l'atome C2
# signifiant le site de liaison à l'atome C3
R⁶ représentant hydrogène, (C₁-C₄)-alkyle ou allyle
(C₁-C₄)-alkyle pouvant être substitué par un substituant choisi dans le groupe formé par hydroxycarbonyle, (C₁-C₄)-alcoxycarbonyle et amino
puis on transforme les composés obtenus des formules (I-C) et (I-D) le cas échéant avec (i) les solvants et/ou (ii) les bases ou les acides correspondants en leurs solvates, sels et/ou solvates des sels.

6. Composé de formule (I), tel que défini dans l'une quelconque des revendications 1 à 4, destiné à être utilisé dans le traitement et/ou la prévention de maladies.

7. Composé selon la revendication 6, destiné à être utilisé dans un procédé pour le traitement et/ou la prophylaxie du diabète, du syndrome métabolique et des dyslipidémies.

8. Utilisation d'un composé de formule (I), tel que défini dans l'une quelconque des revendications 1 à 4, pour la préparation d'un médicament destiné au traitement et/ou à la prévention de maladies coronariennes, du syndrome coronarien aigu, de l'angine de poitrine, de l'insuffisance cardiaque, de l'infarctus du myocarde et de la fibrillation auriculaire.

9. Utilisation selon la revendication 8 pour la préparation d'un médicament destiné au traitement et/ou à la prévention de maladies coronariennes, du syndrome coronarien aigu, de l'angine de poitrine, de l'insuffisance cardiaque, de l'infarctus du myocarde, de la fibrillation auriculaire et de l'hypertonie.

10. Utilisation d'un composé de formule (I), tel que défini dans l'une quelconque des revendications 1 à 4, pour la préparation d'un médicament destiné au traitement et/ou à la prévention du diabète, du syndrome métabolique et des dyslipidémies.

11. Médicament contenant un composé de formule (I), tel que défini dans l'une quelconque des revendications 1 à 4, en combinaison avec un adjuvant inerte, non toxique, pharmaceutiquement approprié.

12. Médicament, contenant un composé de formule (I), tel que défini dans l'une quelconque des revendications 1 à 4, en combinaison avec une ou plusieurs autres substances actives choisies dans le groupe formé par les substances actives modifiant le métabolisme des graisses, les antidiabétiques, les substances actives abaissant la tension artérielle et les agents à action antithrombotique.

13. Médicament selon la revendication 11 ou 12 pour le traitement et/ou la prévention de maladies coronariennes, du syndrome coronarien aigu, de l'angine de poitrine, de l'insuffisance cardiaque, de l'infarctus du myocarde, de la fibrillation auriculaire et de l'hypertonie.

14. Médicament selon la revendication 11 ou 12 pour le traitement et/ou la prévention du diabète, du syndrome métabolique et des dyslipidémies.
